# EUROPEAN PATENT SPECIFICATION

(11) **EP 0 626 008 B1**
(45) Date of publication and mention of the grant of the patent: **29.12.2004**
(21) Application number: 93903565.5
(22) Date of filing: 15.01.1993
(51) Int. Cl.: A61K 39/39, A61K 39/12

(54) **VACCINE CONTAINING ACEMANNAN AS AN ADJUVANT**
IMPFSTOFF, DER ACEMANNAN ALS ADJUVANS ENTHÄLT
VACCIN CONTENANT DE L'ACEMANNANE COMME ADJUVANT

(30) Priority: 17.01.1992 US 822530
(43) Date of publication of application: 30.11.1994
(73) Proprietor: Wyeth Holdings Corporation, Madison, NJ 07940 (US)
(72) Inventor: NORDGREN, Robert, M., Lakeville, MN 55044 (US)
(74) Representative: VOSSIUS & PARTNER
(86) International application number: PCT/US1993/000450
(87) International publication number: WO 1993/014195

(56) References cited:
- EP-A- 0 173 997
- EP-A- 0 282 179
- EP-A- 0 334 530
- EP-A- 0 377 842
- EP-A- 0 423 869
- WO-A-90/01253
- WO-A-90/15596
- WO-A-90/15623
- WO-A-91/04749
- WO-A-93/08810
- US-A- 5 106 616
- CHEMICAL ABSTRACTS, vol. 116, no. 3, 20 January 1992 Columbus, Ohio, US; abstract no. 15533, CHINNAH, ANTHONY D. 'Evaluation of the antiviral, adjuvant, and immunomodulatory effects of a.beta.-(1,4)-linked polymannose ( acemannan )' & (1990) 172 PP. AVAIL.: UNIV. MICROFILMS INT., ORDER NO. DA9118206 FROM: DISS. ABSTR. INT. B 1991, 52(2), 694, 1990
- PROG. CLIN. BIOL. RES. (1984), 161(CHEM. REGUL. IMMUN. VET. MED.), 443-56 CODEN: PCBRD2;ISSN: 0361-7742, 1984 DI LUZIO, N. R. ET AL 'The role of glucan in the prevention and modification of microparasitic diseases'
- VACCINE 10 (8). 1992. 551-557. CODEN: VACCDE ISSN: 0264-410X, CHINNAH A D ET AL 'ANTIGEN DEPENDENT ADJUVANT ACTIVITY OF A POLYDISPERSED BETA-1 4-LINKED ACETYLATED MANNAN ACEMANNAN.'
- Journal of Virology, Volume 65, No. 3, issued March 1991, J.L. CANTELLO et al., "Isolation of a Marek's Disease Virus (MDV) Recombinant Containing the lacZ Gene of Escherichia coli Stably Inserted Within the MDV US2 Gene", pages 1584-1588, see entire document.
- Journal of General Virology, Volume 69, issued September 1988, F.S.B. KIBENGE et al., "Biochemistry and Immunology of Infectious Bursal Disease Virus", pages 1757-1775, see entire document.
- Virology, Volume 126, issued May 1983, V. GOUVEA et al., "In Vitro Characterization of an Avian Reovirus Vaccine Strain", pages 240-247, see entire document.
- Journal of General Virology, Volume 69, issued July 1988, W.L. MENGELING et al., "Size and Antigenic Comparisons Among the Structural Proteins of Selected Autonomous Parvoviruses", pages 825-837, see entire document.
- Journal of Virology, Volume 61, No. 12, issued December 1987, C.C. MARCHIOLI et al., "Evaluation of Pseudorabies Virus Glycoprotein gp50 as a Vaccine for Aujeszky's Disease in Mice and Swine: Expression by Vaccinia Virus and Chinese Hamster Ovary Cells", pages 3977-3982, see entire document.
- Virology, Volume 183, issued February 1991, J.A. FELLER et al., "Isolation and Molecular Characterization of the Swinepox Virus Thymidine Kinase Gene", pages 578-585, see entire document.
- Proceedings of the National Academy of Sciences, USA, Volume 86, issued April 1989, R.A. OLMSTED et al., "Molecular Cloning of Feline Immunodeficiency Virus", pages 2448-2452, see entire document.

## Description

### Background of the Invention

One approach to controlling the spread of viral disease is to administer modified live viruses as vaccines. However, such vaccines may not be 100% effective. Addition of an adjuvant to a vaccine will enhance the immunogenicity of the vaccine and thereby increase its protective capacity.

Marek's disease is of serious concern in the poultry industry. Infection with Marek's Disease Virus can cause a fatal lymphoproliferative disorder and result in significant losses in major production parameters including, but not limited to, condemnations due to leukosis. Other indices of production including mortality, feed conversion and total cost per chick can also be affected by severe outbreaks of the disease. Control of the disease has been attempted using modified live virus vaccine, primarily a modified live Herpesvirus of Turkeys (Marek's Disease Virus serotype III) vaccine.. Although vaccination with Herpesvirus of turkeys (HVT) is considered highly effective in preventing Marek's Disease, some loss continues to occur in low level condemnations due to field challenge, immunosuppression and overt disease due to very virulent Marek's Disease challenge (vvMD). Control of very virulent Marek's Disease has been attempted with bivalent Marek's Disease virus serotype II and III vaccines. However, use of these vaccines is associated with an increase in lymphoid leukosis in certain breeds of birds (Bacon et al., 1989).

Adjuvant-containing vaccine with improved protective capacity would help to eliminate the losses that continue to occur despite the use of presently existing Marek's Disease vaccines and would therefore be of great value in the poultry industry.

The leaf of the Aloe barbadensis plant (Aloe vera) contains large amounts of a β-(1,4) linked acetylated mannan known as acemannan, a water-soluble polymer of about one million kDa. Preparation of acemannan is disclosed by U.S. Patent Nos. 4,735,935 (April 5, 1988), 4,851,224 (July 25, 1989), 4,917,890 (April 17, 1990), 4,957,907 (September 18, 1990), 4,959,214 (September 25, 1990) and 4,966,892 (October 30, 1990). Use of acemannan as an adjuvant to improve the protective capacity of a vaccine has not previously been described.

The present invention provides a Herpesvirus of Turkeys vaccine containing an acemannan adjuvant, a modified live virus vaccine containing an acemannan adjuvant and a modified live virus vaccine containing a complex carbohydrate adjuvant.

### Summary of the Invention

This invention provides a Herpesvirus of Turkeys vaccine which comprises per dose an effective immunizing amount of a modified live Herpesvirus cf Turkeys, an acemannan in an amount effective to enhance the immunogenicity of the Herpesvirus of Turkeys and a suitable carrier. This invention also provides a vaccine which comprises per dose an effective immunizing amount of a modified live virus, an acemannan in an amount effective to enhance the immunogenicity of the modified live virus and a suitable carrier. This invention further provides a vaccine which comprises per dose an effective immunizing amount of a modified live virus, a complex carbohydrate in an amount effective to enhance the immunogenicity of the modified live virus and a suitable carrier wherein said complex carbohydrate is selected from mannan, glucan and levan. A method of immunizing a chick against Marek's Disease and methods of immunizing an animal against viral disease are also provided by this invention.

### Detailed Description of the Invention

This invention provides a Herpesvirus of Turkeys vaccine which comprises per dose an effective immunizing amount of a modified live Herpesvirus of Turkeys (HVT), an acemannan in an amount effective to enhance the immunogenicity of the Herpesvirus of Turkeys and a suitable carrier. For the purposes of this invention, an "effective immunizing amount" of a modified live Herpesvirus of Turkeys is any amount of the modified live Herpesvirus of Turkeys effective to confer immunity upon a fowl. In the practice of this invention, the "effective immunizing amount" of the modified live Herpesvirus of Turkeys is desirably an amount greater than, about 1000 plaque forming units. Preferably, the effective immunizing amount of the modified live Herpesvirus of Turkeys is an amount greater than about 3000 plaque forming units.

It is well known that Herpesvirus of Turkeys is a cell-associated virus. Accordingly, the HVT vaccine of this invention may comprise an effective immunizing amount of the modified live Herpesvirus of Turkeys in Herpesvirus of Turkeys-infected cells. Desirably, the effective immunizing amount of the modified live Herpesvirus of Turkeys is the amount present in about 20,000 HVT-infected cells. Typically, Herpesvirus of Turkeys-infected cells are suspended in a cell culture medium suitable for maintaining the cells in a viable state.

For the purposes of this invention, the "modified live Herpesvirus of Turkeys" may be any avirulent or attenuated Herpesvirus of Turkeys which is capable of inducing an immune response in an animal vaccinated with the virus, for example, the avirulent Herpesvirus of Turkeys strain FC126, available from the American Type Culture Collection (ATCC Accession No. VR 584B).

As stated above, the Herpesvirus of Turkeys vaccine of this invention comprises "an acemannan". As disclosed in U. S. Patent No. 4,851,224, acemannan is a polydispersed polysaccharide compound, with at least 73%. of acemannan polymers having a molecular weight greater than 100,000 daltons. U. S. Patent No. 4,957,907 discloses that acemannan polymers are made up of β(1→4)-linked mannosyl units and that the acetyl groups are linked to the polymer through an oxygen atom, the degree of acetylation being approximately 0.8 acetyl group/monomer, as determined by the alkaline hydroxamate method of Hestrin (J. Biol. Chem. , 180: 240 (1949)). Further disclosed is neutral sugar linkage analysis which indicates that attached to the polymer, probably through an α (2→6) linkage, is a D-galactopyranose residue in the ratio of one for approximately every seventy sugars. The ratio of mannose to galactose of 20:1 indicates that the galactose units are also linked together, primarily by β-(1→4) glycosidic bonds. The chemical structure of an acemannan polymer, as disclosed by U. S. Patent No. 4,957,907, is as follows:

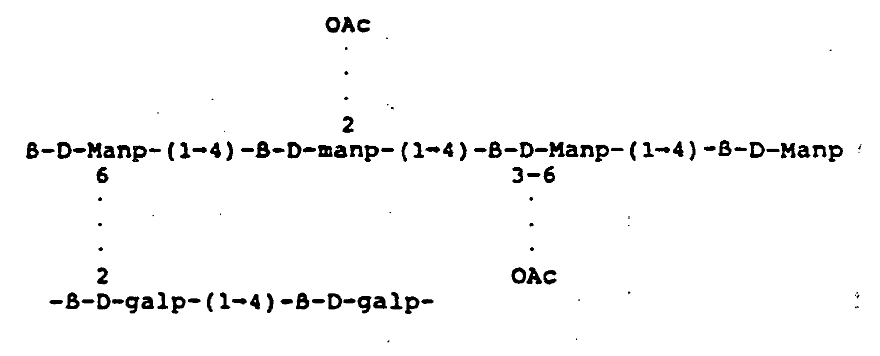

The acemannan may be an extract from the leaf of the Aloe barbadensis plant. However, the term "acemannan" as used herein encompasses not only this extract, but acemannan derived by any method, including chemical synthesis or tissue culture production.

In the practice of this invention, the "effective amount" of the acemannan is typically an amount greater than about 50 micrograms, desirably an amount from about 50 micrograms to about 1000 micrograms. More desirably, the effective amount of the acemannan is an amount from about 50 micrograms to about 150 micrograms. In the presently preferred embodiment of this invention, the effective amount of the acemannan is an amount about 100 micrograms.

Suitable carriers for a vaccine in accordance with the practice of this invention may be any of a number of aqueous buffers well known to those skilled in the art. Presently preferred aqueous buffers are phosphate buffers, e.g., the phosphate buffer described in the examples which follow.

This invention also provides a method of immunizing a chick against Marek's Disease which comprises administering to the chick at one day of age a dose of the Herpesvirus of Turkeys vaccine of this invention. It is presently preferred that the vaccine be administered by subcutaneous injection or by intramuscular injection.

This invention further provides a vaccine which comprises per dose an effective immunizing amount of a modified live virus, an acemannan in an amount effective to enhance the immunogenicity of the modified live virus and a suitable carrier. For the purposes of this invention, an "effective immunizing amount" of a modified live virus is .any amount of the modified live virus effective to confer immunity upon an animal. Methods of determining an effective immunizing amount of a modified live virus are well known to those skilled in the art or can be readily determined without undue experimentation. In the practice of this invention, the effective immunizing amount of the modified live virus is typically an amount greater than about 1,000 plaque forming units. For the purposes of this invention, the "modified live virus" may be any avirulent or attenuated virus which is capable of inducing an immune response in an animal vaccinated with the virus.

In one embodiment of this invention, the modified live virus may be a modified live avian virus. Suitable avian viruses include modified live Marek's Disease viruses, such as Herpesvirus of Turkeys strain FC126; or a Marek's Disease Virus serotype I, e.g., strain R2-23 or CVI-988 strain; or a Marek's Disease Virus serotype II, e.g., strain 301-B1. Suitable viruses also include modified live Newcastle Disease viruses, e.g., Newcastle Disease Virus strain B1B1 or LaSota strain. Suitable avian viruses further include modified live Infectious Bursal Disease viruses, e.g., Infectious Bursal Disease Virus Lukert Strain (Bursine 2) or 2512 strain. Suitable avian viruses still further include modified live Infectious Bronchitis viruses, e.g., Infectious Bronchitis Virus Mass., Conn. or Ark. strains. Suitable avian viruses yet further include modified live Avian reoviruses, e.g., Avian Reovirus strain 1133.

In another embodiment of this invention, the modified live virus may be a modified live swine virus, e.g., a modified live pseudorabies virus, coronavirus or parvovirus.

In still another embodiment of this invention, the modified live virus may be a modified live feline virus, e.g., a modified live Feline Leukemia Virus, Feline T-cell Leukemia Virus or Feline Immunodeficiency Virus.

In yet another embodiment of this invention, the modified live virus may be a modified live canine virus, e.g., a modified live canine coronavirus, parvovirus or distemper virus.

As stated above, the vaccine of this invention comprises "an acemannan". The acemannan may be an extract from the leaf of the Aloe barbadensis plant. However; the term "acemannan" as used herein encompasses not only this extract, but acemannan derived by any method, including chemical synthesis or tissue culture production. In such vaccines, the effective amount of the acemannan is typically an amount greater than about 50 micrograms, desirably an amount from about 50 micrograms to about 1,000 micrograms.

This invention also provides such a vaccine further comprising a second modified live virus. The first modified live virus and the second modified live virus may be modified live avian viruses. In one embodiment of this invention, the first modified live avian virus may be a modified live Herpesvirus of Turkeys and the second modified live avian virus may be a modified live Marek's Disease Virus serotype I, Marek's Disease Virus serotype II, Newcastle Disease Virus, Infectious Bronchitis Virus, Infectious Bursal Disease virus or Avian Reovirus. In another embodiment of this invention, the first modified live avian virus may be a modified live Marek's Disease Virus serotype I and the second modified live avian virus may be a modified live Marek's Disease Virus serotype II, Newcastle Disease Virus, Infectious Bronchitis Virus, Infectious Bursal Disease Virus or Avian Reovirus. In still another embodiment of this invention, the first modified live avian virus may be a modified live Marek's Disease Virus serotype II, and the second modified live avian virus may be a modified live Newcastle Disease Virus, infectious Bronchitis Virus, Infectious Bursal Disease Virus or Avian Reovirus. In yet another embodiment of this invention, the first modified live avian virus may be a modified live Newcastle Disease Virus and the second modified live virus may be a modified live Infectious Bronchitis Virus, Infectious Bursal Disease Virus or Avian Reovirus. In a further embodiment of this invention, the first modified live avian virus may be a modified live Infectious Bronchitis Virus and the second modified live avian virus may be a modified live Irfectious Bursal Disease Virus or Avian Reovirus. In a still further embodiment of this invention, the first modified live avian virus may be a modified live Infectious Bursal Disease Virus and the second modified live avian virus may be a modified live Avian Reovirus.

The first modified live virus and the second modified live virus may also be modified live swine viruses, modified live feline viruses or modified live canine viruses.

This invention further provides such a vaccine further comprising a third modified live virus.

This invention provides a method of immunizing an animal against a viral disease which comprises administering to the animal a dose of the vaccine of this invention. The animal may be a day of age fowl, e.g., a day of age chicken, turkey, duck or quail. The animal may also be a porcine. The animal may further be a feline. The animal may still further be a canine. The appropriate age at which to administer a vaccine to an animal, and the intervals at which to administer booster vaccine, if necessary, is well known to those skilled in the art or can readily be determined without undue experimentation. The vaccine may be administered orally, by eye drops, subcutaneous injection or intramuscular injection.

This invention provides a vaccine which comprises per dose an effective immunizing amount of a modified live virus, an amount of a complex carbohydrate effective to enhance the immunogenicity of the modified live virus and a suitable carrier. For the purposes of this invention, an "effective immunizing amount" of a modified live virus is any amount of the modified live virus effective to confer immunity upon an animal. Methods of determining an effective immunizing amount of a modified live virus are well known to those skilled in the art or can be readily determined by rouitne experimentation. In the practice of this invention, the effective immunizing amount of the modified live virus is typically an amount greater than about 1,000 plaque forming units. The modified live virus may be any avirulent or attenuated virus capable of inducing an immune response in an animal vaccinated with the virus. In one embodiment of this invention, the modified live virus is a modified live avian virus, e.g., a modified live Herpesvirus of Turkeys.

As stated above, the vaccine of this invention, comprises a complex carbohydrate. Methods of determining an "effective amount" of a complex carbohydrate are well known to those skilled in the art or can be readily determined by routine experimentation. Typically, the effective amount of the complex carbohydrate is an amount greater than about 50 micrograms, desirably an amount from about 50 micrograms to about 1,000 micrograms. In one embodiment of this invention, the complex carbohydrate may be a mannan, e.g., a β-(1→4)linked mannan. Preferably, the β-(1→4)linked mannan is acemannan. In another embodiment of this invention, the complex carbohydrate may be a glucan, e.g., a β-(1→3)-linked glucans. A β-(1→3)-linked glucan useful in the practice of this invention may be a lentinan. Glucans are polysaccharide compounds with polymers made up of glucose units. In still another embodiment of this invention, the complex carbohydrate is a levan, e.g., a β-(2→6)-linked lvean. A β-(2→6)-linked levan useful in the practice of this invention may be a levan from Aerobacter levanicum.

This invention also provides a method of immunizing an animal against viral disease which comprises administering to the animal a dose of the vaccine of this invention. The animal may be a fowl, e.g., a chicken, turkey, duck or quail.

In the practice of this invention, the effective amount of the acemannan may be readministered to the animal at an interval after the preceding administration. Appropriate intervals between administrations of acemannan are well known to those skilled in the art or can readily be determined without undue experimentation.

This invention will be better understood from the Examples which follow. However, those skilled in the art will readily appreciate that the specific examples detailed are only illustrative of the invention as described more fully in the claims which follow thereafter.

### Examples

### Example 1

### Preparation of Herpesvirus of Turkeys Vaccine

Herpesvirus of Turkeys (HVT) was obtained from the American Type Culture Collection (ATCC), 12301 Parklawn Drive, Rockville, Maryland 20852, as the eleventh passage in duck embryo fibroblasts. The FC#126 Herpesvirus of Turkeys (presently available as ATCC Accession No. VR 584B) may be the strain used to prepare the Marek's Disease vaccine. The vaccine contains cell culture material containing 100% HVT and stabilizer.

Each Master Seed ("X") virus is identified by the characteristic Cytopathogenic Effect (CPE) in chicken embryonic fibroblast (CEF) cells which contain foci of rounded, refractile and destroyed cells. The Master Seed ("X") is specifically identified by an agar gel diffusion test or by specific direct reaction of infected cells with fluorescein-conjugated anti-Herpesvirus of Turkeys serum.

The Herpesvirus of Turkeys is avirulent.

Each batch of CEF cells used in the preparation of the Master Seed virus (X") has been tested in accordance with 9 C.F.R. 113.26(a)(2), 113.26(a)(3), 113.30 and 113.51(c). The Master Seed has been tested in accordance with 9 C.F.R. 113.300 and 113.330, and specifically in accordance with 113.27, 113.28, 113.31, 113.34 and 113.37. The Master Seed has been safety tested in accordance with 9 C.F.R. 113.330.

The Master Seed ("X") is a suspension of viable Herpesvirus of Turkeys (HVT)-infected chicken embryo fibroblasts (CEF). The suspension is frozen and stored in ampules at -100°C or colder in the vapor phase of liquid nitrogen or in liquid nitrogen.

The Master Seed is the 16th passage, and is designated as the "X" passage. The seed passage range will be between the 16th and 19th passages in cell culture. The vaccine virus range will be between the 17th and 20th passages. Following is the schematic schedule of seed passage:

| **SCHEMATIC SCHEDULE OP SEED PASSAGE** | | |
|---|---|---|
| Received: | | American Type Culture Collection, 12301 Parklawn Drive, Rockville, MD 20857. |
| Received: | 11^{th} Passage | (Strain FC #126) |
| | Production Seed | |
| Master Seed ("X") | 16^{th} Passage | |
| ("X+1") | 17^{th} Passage | |
| ("X+2") | 18^{th} Passage | |
| ("X+3") | 19^{th} Passage | |
| | Production Seed | |
| ("X+4") | 20^{th} Passage | Vaccine Virus |

All medium components of animal origin that are not sterilized by autoclaving will be tested for contaminants specifically in accordance with 9 C.F.R. 113.53. The culture medium for cell cultures is identical for the propagation of both seed and production cultures. The growth medium is Eagle's Minimum Essential Medium with Earle's Salts and L-glutamine.

The growth medium is supplemented with 5% tryptose phosphate broth and 1% to 5% fetal calf serum. The serum may be heat-treated for 30 minutes at 56°C. The serum is obtained sterile from commercial manufacturers and meets the requirements of 9 C.F.R. 113.53. The serum may be sterilized by filtration, by gamma-irradiation or by treatment with beta-propiolaetone. Serum sterilized with beta-propiolactone is tested for bacteria and fungi in accordance with 9 C.F.R. 113.26, Mycoplasma in accordance with 9 C.F.R. 113.28 and Adventitious virus in accordance with 9 C.F.R. 113.53(c). Gamma-irradiated serum is tested for, and found free of, bacteria and fungi in accordance with 9 C.F.R. 113.26, Mycoplasma in accordance with 9 C.F.R. 113.28 and Adventitious viruses in accordance with 9 C.F.R. 113.53, before gamma-irradiation. Serum may be obtained from JRH Biosciences, 13804 West 107th Street, Lenexa, Kansas 66215, or Metrix Company, 4400 Chavenelle Road, Dubuque, Iowa 52001-9673.

The serum may be used as a nutritional supplement in biological growth systems and is stored at 7°C or colder for no more than one year after receipt from the manufacturer.

The chick embryos used in cell cultures are nine to eleven days old. The embryos are taken from eggs secured from specific pathogen free (SPF) flocks. Chicken eggs for live virus vaccine production are secured from flocks that are specifically tested for, and are free of, the following agents: Avian Adeno Virus (AAV); Avian Encephalomyelitis Virus (AEV); Avian Leukosis Virus (ALV) or Rous Sarcoma Antibody A and B (RSV-RAV₁ and RSV-RAV₂); Fowl Pox Virus (FPV); Infectious Bronchitis Virus (IBV), Connecticut Type; Infectious Bronchitis Virus (IBV), Massachusetts Type; Infectious Laryngotracheitis Virus (ILV); Newcastle Disease Virus (NDV); Mycoplasma gallisepticum (MG); Salmonella pullorum - Typhoid; Avian Influenza Virus - Type A; Reovirus; and Infectious Bursal Disease virus (IBDV).

One hundred percent of the birds are bled, between 12 and 20 weeks of age. Blood samples are tested for the above-mentioned diseases, except for Avian Encephalomyelitis Virus (AEV). AEV tests may be conducted after the flock is in production, but before the eggs are used for vaccine production, using 50 embryos per test.

Monitoring is achieved by testing no less than 5% of the flock, at approximately monthly intervals. Testing may be performed by the following laboratories: Solvay Animal Health Inc., Charles City, Iowa; SPAFAS, Inc., Norwich, Connecticut; ISA Laboratories, Ithaca, New York; HY-Vac Laboratory Eggs Company, Cowrie, Iowa; State Laboratory of Minnesota, Minneapolis, Minnesota; State Laboratory of Ohio, Columbus, Ohio; and Lasher Associates, Inc., Millsboro, Delaware.

Cell suspensions are prepared using nine- to twelve-day embryonated SPF eggs of Gallus gallus, phosphate buffered saline (PBS), sterile trypsin and Minimum Essential Medium (MEM) or Media 199 Growth Medium according to the following procedure. Medium 1199 is a standard growth medium, readily available to those skilled in the art.

The eggs are disinfected with an alcohol-iodine solution. when dry, the eggs are placed under a laminar flow hood and the shell is cracked with sterile forceps. The embryos are aseptically removed from their membranes, and are pooled into sterile petri dishes. The heads are removed with modified sterile surgical scissors. The torsos and heads are pooled, distributed into sterile containers and then washed with PBS containing no antibiotics. The washed embryos are then transferred to trypsinizing flasks for a final wash with PBS. After the final wash, no more than 0.4% trypsin in PBS containing antibiotics are added to the flasks. The suspension is agitated with a magnetic stirrer to further disperse the cells.

The cells are extracted for cumulative periods of no more than 100 minutes from embryo torsos, and for cumulative periods of no more than 40 minutes from embryo heads. The cells from these extractions are centrifuged at no less than 250 x g for ten minutes in one-liter centrifuge tubes containing no more than 5% fetal bovine serum (FBS). The supernatant is decanted, and the cells are suspended in growth medium at a maximum ratio of approximately 1:5 cells to medium. The resulting suspensions are pooled into a common container, and are agitated with a magnetic stirrer. After thorough mixing, the cell count is determined. Representative samples are collected after the first wash of embryo torsos and heads in PBS and tested in accordance with 9 C.F.R. 113.30 and 9 C.F.R. 113.51. CEF cells are used to support virus growth in the production of vaccine or in testing systems for the assay of virus.

Cells are dispersed into the growth medium described above at a final concentration of approximately 0.5 to 5.0 x 10⁶ cells per ml. The cells are planted in sterile roller bottles, 85 ml to 500 ml of medium per bottle, and are incubated at 37°C ± 2°C for 20 to 72 hours. Cells may be re-fed with fresh medium if kept longer than 46 hours. Bottles that are contaminated, have atypical monolayers or an atypical pH are discarded.

Cell cultures are propagated in roller bottles ranging in size from approximately 2,000 ml to 9,000 ml. The bottles are either a soft glass, a type I borosilicate glass, or are sterile commercial plastic bottles. The glass bottles are steam-sterilized for a minimum of five minutes at no less than 132°C ± 2°C, for a minimum of 15 minutes at no less than 121°C ± 2°C or are dry-heat-sterilized for a minimum of one hour at no less than 160°C ± 2°C. The bottles are used within two weeks after sterilization or are re-sterilized. The bottles will be sealed with a rubber-lined screw-cap or with a rubber closure. The closures and/or screw-caps are sterilized.

The Master Seed ("X") is stored in ampules at -100°C or colder in the vapor phase of liquid nitrogen, or are stored in liquid nitrogen as viable Herpesvirus of Turkeys (HVT)-infected cells.

Preparation of cell suspensions for seeding or inoculating first requires that stock seed cultures be removed from the liquid nitrogen and rapidly thawed. Seed cultures are aseptically withdrawn from the thawed ampules and are diluted to no less than 500 plaque forming units (pfu's) per ml for inoculation onto chicken embryo fibroblast (CEF) monolayers in roller bottles.

Subsequent seed harvests' are accomplished by removing the cells from the roller bottles with 0.05% to 0.25% trypsin.

Trypsin solutions are prepared as follows:

| Solution No. 1: | |
|---|---|
| Trypsin 1:250, T. C. Grade Phosphate Buffered Saline (PBS) Quantity | 250.0 grams |
| sufficient (q.s.) | 10,000.0 ml |

The trypsin is slurried with a small amount of warm phosphate buffered saline (PBS). Warm PBS is added to 25% of the final volume. The solution is stirred at a moderate rate of speed to avoid foaming. The remaining volume is made up with additional PBS, and the solution is mixed well.

| Solution No. 2: | |
|---|---|
| Trypsin 1:250, (10X) Commercially-Prepared Solution -- β-propiolactone-treated (BPL-treated) | 2,000.0 ml |
| Sodium Chloride, U.S.P. | 320.0 grams |
| Potassium Chloride, U.S.P. | 16.0 grams |
| Dextrose Hydrate | 80.0 grams |
| Sodium Bicarbonate | 28.0 grams |
| Ethylenediaminetetraacetic Acid (EDTA) .. | 20.0 grams |
| Deionized Super Q H₂O ..... quantity sufficient (q.s.) | 10,000.0 ml |

Each dry ingredient is mixed separately with deionized water and is warmed. The solutions are then combined and the trypsin and ethylenediaminetetraacetic acid (EDTA) solutions are added. The solution is stirred at a moderate rate of speed until well mixed.

Trypsin solutions 1 and 2 may be sterilized by final filtration through a sterile 0.2-micron filter.. The sterilized solution is filtered and dispensed into sterile Type I borosilicate glass bottles at the rate of 25 ml to 500 ml per bottle. The bottles are sealed with rubber-lined screw-caps. Solutions sterilized in this manner are tested for bacteria and fungi in accordance with 9 C.F.R. 113.26, Mycoplasma in accordance with 9 C.F.R. 113.28 and pathogens by chicken embryo inoculation in accordance with 9 C.F.R. 113.37. Each lot of trypsin powder is tested for Porcine Parvovirus in accordance with 9 C.F.R. 113.53(d). Trypsin solutions may also be sterilized using β-propriolactone. Screw-capped Erlenmeyer flasks of a size four times the volume of the material to be treated are used. A salt solution, e.g., Dulbecco's Balanced Salt Solution, Mg⁺⁺ and Ca⁺⁺ free, containing 2.5% trypsin is made up. β-propriolactone is put into solution by violent shaking and diluted to a 10% concentration with ice cold distilled water, under a protective hood using a propipetter and protective clothing. The diluted BPL is added to the trypsin at the rate of 1 ml to 99 ml of cold trypsin to be treated, making a final dilution of 0.1% BPL. The BPL-trypsin is stirred slowly overnight in a walk-in refrigerator, and is frequently checked to prevent frothing, which may hold virus above the inactivant. The treated material may be diluted ten-fold to make a 0.25% trypsin solution for immediate use, or the concentrate may be filtered and stored frozen for later dilution and use. Solutions sterilized with β-propriolactone are tested for bacteria and fungi in accordance with 9 C.F.R. 113.26, Mycoplasma in accordance with 9 C.F.R. 113.28 and pathogens by chick embryo inoculation, in accordance with 9 C.F.R. 113.37. Solutions sterilized in this manner are tested for Porcine Parvovirus in accordance with 9 C.F.R., or a protocol established by the manufacturer.

Solution No. 3 is a commercially prepared Trypsin 1:250 (10X) solution which may be obtained from Gibco Laboratories, 3175 Staley Road, Grand Island, New York 14072. The trypsin meets the requirements of 9 C.F.R. 113.53. Solution No. 4 is a commercially prepared 1:250-ethylenediaminetetraacetic acid (EDTA) (10X)-treated, gamma-irradiated solution which may be obtained from Sigma Chemical Company, Post Office Box 14508, St. Louis, Missouri 63178. Gamma-irradiated trypsin solutions are tested for bacteria and fungi in accordance with U.S.P. XXI, Mycoplasma by the Barile method and Porcine Parvovirus in accordance with 9 C.F.R. 113.53. Solution No. 5 is a commercially prepared Trypsin 1:250 (10X) BPL-treated solution which may be obtained from JRH Biosciences, 13804 West 107th Street, Lenexa, Kansas 66215.

The trypsin concentrates (Solutions 1, 3 and No 4) are diluted to the appropriate concentration with sterile phosphate buffered saline (PBS) and are used for the dispersion of cell cultures. The trypsin concentrate (Solution No. 2) is diluted to the appropriate concentration with Super Q H₂O and is used for the dispersion of cell culture. The trypsin concentrate (Solution No. 5) is used in the preparation of solution No. 2. The various trypsin concentrates are stored at -20°C, or colder, for no more than 12 months.

Fetal calf serum may be added to the harvested cells to inactive the trypsin before centrifuging. Harvested cells are centrifuged for 10 to 20 minutes. The supernatant is decanted, and the cells are resuspended at a volume:volume ratio ranging from 1:40 to 1:80 of packed cells to PBS. The phosphate buffered saline is supplemented with 4% fetal calf serum and antibiotics.

The seed virus suspension is inoculated aseptically onto CEF monolayers at a rate of two to three ml per roller bottle. The seed virus volume is expanded by passage of infected cells in the range of 1:5 to 1:30. The inoculation of seed and production medium is identical. The inoculated monolayers may be supplemented with additional primary CEF, when available, as follows: primary CEF cells are prepared as described above; the primary CEF cells are then added as a concentrate to Herpesvirus of Turkeys (HVT)-inoculated roller bottles at a rate of 0.5 to 5.0 x 10⁸ cells per bottle, depending upon the number of cells available and the cell concentrate is added at 18 to 24 hours post-inoculation. The cell concentrate is tested for Salmonella in accordance with 9 C.F.R. 113.30 and Adventitious agents in accordance with 9 C.F.R. 113.51.

The Herpesvirus of Turkeys-infected CEF monolayers are incubated at 37°C ± 2°C for 24 to 72 hours. Characteristic cytopathogenic effect (CPE), as determined by experience and observation, must be evident in 80% or more,of the cells before harvest. Ail containers not showing characteristic CPE, and those with bacterial contamination or atypical cell monolayers, are discarded.

Bottles are selected at random and are examined microscopically for characteristic CPE and for evidence of contamination. The minimum to maximum period of time elapsing from the time of inoculation until harvest is 24 to 72 hours. Preferably, harvesting is done at 48 hours post-inoculation.

After centrifugation of cell suspensions, the supernatant culture fluid is discarded and the cells are detached from the glass surface with 0.05% to 0.25% trypsin. Cells are ,then pooled from the roller bottles into one-liter centrifuge bottles. Fetal calf serum may be added to the cells to inactivate the trypsin. The pooled cells are centrifuged at no less than 250 × g for no less than f ive to fifteen minutes. After the supernatant-trypsin solution is discarded, the packed cells are resuspended in sterile Minimal Essential Medium or Medium #199 with Earle's Buffered Saline Solution (BSS) and 15% fetal calf serum. The volume of this suspension is 40% to 80% of the final volume of the vaccine. Only monolayers that show characteristic CPE and are free of any indication of contamination, such as abnormal cells, pH, turbidity, odor, and molds shall be harvested. All material not used for vaccine production shall be disposed of in accordance with Veterinary Services Memorandum No. 800.56.

The HVT-infected cells are maintained in a viable state by controlled freezing in the following medium:

| Ingredients | | Per ten liters |
|---|---|---|
| 1. | Minimum Essential Medium or Medium #199 with Earle's BSS with 10% glutamine | 7,400 to 7,480 ml |
| 2. | Tryptose Phosphate Broth (TPB) | 500 ml |
| 3. | NaHCO₃ (10%), Reagent grade | 20 to 100 ml |
| 4. | Fetal calf serum | 1,500 ml |
| 5. | Dimethyl sulfoxide (DMSO) | 500 ml |

Medium #199 is a standard medium readily available to practitioners in the art. The medium is prepared for use within 72 hours. The dimethyl sulfoxide is sterilized at 121±3°C for fifteen minutes.

The product is standardized by first adjusting the cell suspension cell to a final concentration of no less than 1 x 10⁷ cells per ml, as determined by standard cell counting methods, such as by hemacytometer or by an electric particle counter. Cell viability is estimated by the use of vita stains, e.g., Trypan Blue or Erythrosin B.

A serial is assembled by first passing suspended cells through sterile lint-free gauze or a sterile stainless steel screen. While the cell suspension is mechanically agitated, a mixture of freeze medium is slowly added to achieve the described concentration of cells in the batch. The cell suspension is maintained at refrigerated temperatures at all times. An example of assembling a serial, based on a 20,000-ml batch, is as follows:

| | | |
|---|---|---|
| 1) | HVT-infected cells | 2 x 10¹¹ |
| 2) | Minimum Essential Medium (Eagle's) or Media #199 with L-glutamine | 14,800 ml to 14,960 ml |
| 3) | Tryptose Phosphate Broth | 1,000 ml |
| 4) | NaHCO₃, 10% Reagent Grade | 40 ml to 200 ml |
| 5) | Fetal Calf Serum | 3,000 ml |
| 6) | Dimethylsulfoxide (DMSO) | 1,000 ml |
| TOTAL VOLUME | | 20, 000 ml ± 3% |

The average serial volume will be approximately 20,000 ml. The maximum volume of a serial will be 40,000 ml.

The fill volume is 2.0 ± 0.2 ml per 500- to 3,000-dose ampule, and 1.0 ± 0.1 ml per 500-dose ampule. Vaccine is dispensed into two-ml Type I borosilicate glass ampules that have been prescored and sterilized. Acemannan adjuvant is dispensed into 20 or 50 ml Type I borosilicate bottles using 20mm rubber or butyl stoppers.

The diluent shall be manufactured, tested and sold in accordance with Veterinary Services Memorandum No. 800.74. A one-liter batch of diluent will be composed of:

| | |
|---|---|
| NZ Amine | 14.00 g |
| Sucrose, food grade | 50.00 g |
| K₂HPO₄ · 3H₂O (Potassium Phosphate Dibasic) | 1.48 g |
| Phenol Red, A.C.S. | 0.01 g |
| Deionized Water | 1,000.00 ml |

The above-listed ingredients are dissolved in a small volume of deionized water by stirring. The mixture is diluted to final volume with deionized water after the ingredients are in solution and the pH is adjusted to 7.1 or 7.3 with NaOH or HCl. The average batch size of diluent will be between 1,000,000 ml and 2,400,000 ml; the maximum batch will be 3,000,000 ml. No preservatives are added to the diluent.

The vaccine, under constant agitation, is aseptically filled into sterile glass ampules which are flame-sealed and placed in canes for storage. Filling and sealing is done by an automatic filling machine, and is carried out at 10°C ± 5°C. The ampules of vaccine are frozen by cooling at a constant rate of about 1°C per minute in a controlled freezing chamber. The ampules are stored at approximately -100°C or colder in the vapor phase of liquid nitrogen or in liquid nitrogen.

Each dose of vaccine in the final container will contain no less than 20,000 cells, counted as described above. Containers of vaccine are maintained in liquid nitrogen during shipment and storage, while adjuvant is maintained at room temperature during shipment and storage.

Each batch of cells is tested for Salmonella in accordance with 9 CFR 113.30 and Adventitious agents in accordance with 9 CFR 113.51. Final container samples of vaccine and adjuvant are tested for Mycoplasma in accordance with 9 CFR 113.28, bacteria and fungi in accordance with 9 CFR 113.27, hemagglutinating viruses in accordance with 9 CFR 113.34 and Adventitious agents in accordance with 9 CFR 113.37. Bulk or final containers samples of vaccine are tested for Avian Lymphoid Leukosis in accordance with 9 CFR 113.31.

Final container samples are tested for safety in accordance with 9 CFR 113.330.

The vaccine is titrated in accordance with 9 C.F.R. 113.330 and by methods generally known to those skilled in the art. The Master Seed ("X") was prepared on January 17, 1986 and Testing of the fourth passage ("X+4") from the Master Seed virus was completed on July 10, 1986. Vaccine is prepared from this fourth passage. Vaccine titer at release shall be no less than 3,000 plaque forming units (pfu's) per bird dose. Vaccine titer throughout the expiration date shall be no less than 1,000 pfu's per bird dose.

Adjuvant moisture, determined in accordance with 9 CFR 113.29, shall not exceed 5%.

Vaccine is packaged in two-ml ampules, five ampules per cane, and one cane per package. Adjuvant is packaged in 20-or 50-ml bottles, 5 bottles per package. Acemannan (Carrisyn™) is supplied by Carrington Laboratories, Irving, Texas. One hundred micrograms of acemannan are supplied with each dose of vaccine and diluent. The acemannan is distributed as a sterile powder or lyophilized in sterile stoppered vials. The acemannan supplied will meet the specifications of Carrington Laboratories for "Bulk Carrisyn Certificate of Analysis" for identification, percent polimannoacetate, anthraquinone concentration and molecular weight. The acemannan is reconstituted with diluent (see above) prior to vaccination. A direction leaflet and sterile diluent shall be provided. Representative samples of vaccine and adjuvant will be collected after freezing for quality assurance testing.

The expiration date of the vaccine shall not exceed 22 months from the date of initiation of potency testing, in accordance with 9 CFR 114.13.

The vac e is to be used for the vaccination of day-old chicks for the prevention of Marek's Disease. Twenty mg. of acemannan may be suspended in 200 ml of the diluent described above, and two ml of thawed vaccine added to this, to produce 1,000 bird doses of vaccine. One 0.2 ml dose of vaccine is to be administered per bird. The vaccine is administered subcutaneously or intramuscularly.

The birds used in these studies were all maternal antibody negative Specific Pathogen Free (SPF) leghorns. Birds were vaccinated at one day of age, then housed in separate negative pressure Horsfall-type plexiglass incubators, fed and watered *ad libitum*, with a minimum of 200-cm² floor space allowed for each bird throughout the period of the test.

### Example 2

### Response of HVT/Acemannan Vaccinated Chicks to Marek's Disease Virus RB1B Challenge Administered at One, Two, Three or Four Days Post-Vaccination

The Herpesvirus of Turkeys vaccine used was diluted to a theoretical dose of 3500 pfu's per 0.2 ml dose in the phosphate buffer described above. Acemannan was supplied by Carrington Laboratories, Irving TX, as a gamma irradiated sterile powder. Acemannan was mixed with the phosphate buffer diluent prior to addition of virus at a 100ug per 0.2ml dose level. Control diluent was the phosphate buffer with no other additions.

Marek's Disease Virus RB1B challenge virus was administered at a theoretical dose of 700 pfu's per bird in an intraperitoneal injection. RB1B, a typical very virulent Marek's disease challenge, was obtained from Dr. Witter at East Lansing Regional Poultry Research Laboratory and was propagated on primary chick kidney cell cultures.

Birds were divided into the following groups of 30 and housed in separate isolators: HVT challenged day 1,2,3 & 4 post-vaccination; HVT & acemannan challenged 1,2,3 & 4 days post-vaccination; acemannan in phosphate buffer diluent challenged 1,2,3 & 4 days post-vaccination; and nonvaccinate, diluent control challenged 1,2,3 & 4 days post-vaccination of the other groups.

At the appropriate day post treatment, vaccinates were injected intraperitoneally with 750 plaque forming units of the RB1B virus and wing banded with consecutively numbered monochrome bands. Following the final challenge, all birds from each challenge period and vaccination group were randomly mixed and placed in isolators in order to maintain a constant background challenge.

Birds were observed for signs of post challenge trauma and posted in the event of death. All groups were observed for the remainder of the 48 day test period, and posted and examined for lesions pathognomonic for vvMD (very virulent Marek's Disease challenge). Mortality due to vvMD, and birds with lesions from vvMD were totaled, and viewed relative to the total incidence in the nonvaccinated birds in order to determine the protective index of the treatment. Marek's Disease immunity is reported as a protective index (PI) where the performance of each group is compared to the infectivity level of the control. Protective Index = (% positive controls) - (% positive vaccinates)/(% positive controls)) x 100.

The results presented in Table 1 (see below) indicate that the challenge administered to birds at this early age was severe, with 100% of all nonvaccinates being affected. The data also indicates that the addition of the acemannan to the HVT had a significant effect on the vaccine, with the day 2, 3 and 4 day post vaccination challenge groups performing significantly better than the HVT alone groups. The results in Table 2 (see below) indicate that the HVT/acemannan treated birds performed better overall, irrespective of the day birds were challenged.

**Table 1**

| **EFFECT OF THE DAY OF CHALLENGE ON THE DAY OF AGE VACCINATED CHICKS** | | | | | | |
|---|---|---|---|---|---|---|
| Group | Day of Challenge | # in group | Non-specific mortality | deaths with tumors | Tumors | P.I.* |
| NonVac | day 1 | 10 | 0 | 5 | 5 | 0.00 |
| Acm | day 1 | 20 | 4 | 6 | 7 | 18.75 |
| HVT | day 1 | 20 | 5 | 9 | 5 | 6.67 |
| HVT/Acm | day 1 | 20 | 3 | 8 | 6 | 17.65 |
| NonVac | day 2 | 10 | 0 | 7 | 3 | 0.00 |
| Acm | day 2 | 20 | 6 | 5 | 5 | 28.57 |
| HVT | day 2 | 20 | 3 | 5 | 7 | 29.41 |
| HVT/Acm | day 2 | 20 | 5 | 2 | 3 | 66.67 |
| NonVac | day 3 | 10 | 2 | 6 | 2 | 0.00 |
| Acm | day 3 | 20 | 5 | 4 | 6 | 33.33 |
| HVT | day 3 | 20 | 1 | 3 | 3 | 68.42 |
| HVT/Acm | day 3 | 20 | 0 | 1 | 2 | 85.00 |
| NonVac | day 4 | 10 | 1 | 4 | 5 | 0.00 |
| Acm | day 4 | 20 | 3 | 6 | 5 | 35.29 |
| HVT | day 4 | 20 | 4 | 3 | 2 | 68.75 |
| HVT/Acm | day 4 | 20 | 2 | 0 | 1 | 94.44 |

| | | | | | | |
|---|---|---|---|---|---|---|
| * P.I. = protective index: % of non-vaccinated challenged birds, less the % of vaccinated challenged birds, divided by the % of non-vaccinated challenged birds. Nonvac = nonvaccinated: Acm = acemannan. Nonspecific mortality refers to dead birds having no observable lesions. | | | | | | |

**Table 2**

| **CUMULATIVE PROTECTION DATA IRRESPECTIVE OF DAY OF CHALLENGE** | | | | | |
|---|---|---|---|---|---|
| Group | # in group | Non-specific mortality | Deaths with tumors | Tumors | P.I.* |
| NonVac | 40 | 3 | 22 | 15 | 0 |
| Acm | 80 | 18 | 21 | 23 | 29.0 |
| HVT | 80 | 13 | 20 | 17 | 44.8 |
| HVT/Acm | 80 | 10 | 11 | 12 | 68.6 |

| | | | | | |
|---|---|---|---|---|---|
| * P.I. = protective index: % of non-vaccinated challenged birds, less the % of vaccinated challenged birds, divided by the % of non-vaccinated challenged birds. Nonvac = nonvaccinated; Acm - acemannan. Nonspecific mortality refers to dead birds having no observable lesions. | | | | | |

These data suggest that addition of acemannan to HVT vaccine at the 3,500 pfu and 100µg per bird dose level, offered significantly improved protection to chicks challenged at 2, 3 or 4 days post-vaccination.

Also of interest is the difference in nonspecific mortality between the various vaccinate groups. While no significant differences occur between the vaccinate groups, there is a clear difference in the percentage of nonspecific mortality between the vaccinates and the nonvaccinates. The experimental design calls for the evaluation of the vaccine by observing the reduction of gross tumors. It may be that the vaccinates are partially protected against frank tumor development, but with this particular vaccine challenge are affected in other ways. If this is the case, review of the data indicates that there was less mortality in the HVT/acemannan group, suggesting that the apparent adjuvant effect of acemannan remains the same or increases.

### Example 3

### Effect of Acemannan on Early, Virulent Challenge After HVT Vaccination

Day of age birds were divided into groups and administered either zero, 250 or 1000 pfus of the modified live HVT and either zero or 100 micrograms (µg) of acemannan (see Table 3, below).

Challenge virus was either the RB1B or JMV strains of Marek's Disease Virus. RB1B, a typical very virulent Marek's disease challenge, was obtained from Dr. Witter at East Lansing Regional Poultry Research Laboratory and was administered in 0.2 ml intraperitoneal doses containing 400 pfu's per dose of the challenged virus. Challenge was either five or twenty one days post vaccination. Birds were monitored for signs of paralysis and mortalities were assessed over a period of 48 to 60 days post-infection. At the end of the observation, all birds were euthanized, and posted, to record signs of gross tumor development, particularly in the heart, liver, kidney, spleen and testes or ovaries. JMV transplantable tumor was obtained from Dr. Sevoian, University of Massachusetts, and was injected intraperitoneally into day of age donor chicks concurrent with vaccination of the test animals. At five days post infection, the JMV chicks were euthanized and livers and spleens were collected, minced in sterile RPMI and then macerated in a Tenbrock^{R} tissue homogenizer. The resulting homogenate was then diluted 3:1 in fresh RPMI and 0.2 ml was injected intraperitoneally into chicks. The chicks were observed for 21 days, during which mortality was recorded.

The data shown in Table 3 suggest that addition of acemannan to HVT vaccine improves the protective capacity of the vaccine as all groups receiving acemannan had a lower percentage of member birds affected by disease and a higher protective index than did the corresponding groups not receiving acemannan-containing vaccine. Protective index compares the performance of each experimental group to the infectivity level of the control.

**Table 3**

| **VACCINATION OF ONE DAY OF AGE CHICKENS WITH MODIFIED LIVE HERPESVIRUS OF TURKEYS AND ACEMANNAN AND VERY VIRULENT MAREK'S DISEASE CHALLENGE** | | | | |
|---|---|---|---|---|
| A. Experimental Design | | | | |
| Group | PFUs | Quantity of Acm(µg) | Challenge (days) | # of Birds |
| 1 | 0 | 0 | RB1B(5) | 14 |
| 2 | 250 | 0 | RB1B(5) | 15 |
| 3 | 250 | 100 | RB1B(5) | 15 |
| 4 | 1000 | 0 | RB1B(5) | 15 |
| 5 | 1000 | 100 | RB1B(5) | 15 |
| 6 | 0 | 0 | RB1B(21) | 20 |
| 7 | 250 | 0 | RB1B(21) | 20 |
| 8 | 250 | 100 | RB1B(21) | 20 |
| 9 | 1000 | 0 | RB1B(21) | 20 |
| 10 | 1000 | 100 | RB1B(21) | 20 |

| B. Results | | | | |
|---|---|---|---|---|
| Group | # of Dead | # with Tumors | % Affected | P.I.* |
| 1 | 0 | 12 | 86 | 0 |
| 2 | 0 | 6 | 40 | 53 |
| 3 | 0 | 3 | 20 | 77 |
| 4 | 0 | 6 | 40 | 53 |
| 5 | 0 | 5 | 33 | 62 |
| 6 | 4 | 17 | 85 | 0 |
| 7 | 0 | 10 | 50 | 41 |
| 8 | 2 | 6 | 40 | 53 |
| 9 | 0 | 10 | 50 | 41 |
| 10 | 0 | 8 | 40 | 53 |

| | | | | |
|---|---|---|---|---|
| * P.I. = protective index: % of non-vaccinated challenged birds, less the % of vaccinated challenged birds, divided by the % of non-vaccinated challenged birds. Acm = acemannan. | | | | |

### Example 4

### Effect of Acemannan on Early, Virulent Challenge After HVT Vaccination

Day of age birds were divided into groups and administered either zero, 625, 1250 or 3750 pfus of the modified live HVT virus and either zero or 100 micrograms of acemannan (see Table 4, below). Challenge was with the RB1B strain of the Marek's disease virus at five days after vaccination, as described in Example 3 (see above).

The data shown in Table 4 suggest that addition of acemannan to HVT vaccine improves the protective capacity of the vaccine. All groups receiving acemannan-containing vaccine had fewer dead member birds, a lower percentage of member birds affected by disease and a higher protective index than did the corresponding groups not receiving acemannan-containing vaccine.

**Table 4**

| **VACCINATION OF ONE DAY OF AGE CHICKENS WITH MODIFIED LIVE HERPESVIRUS OF TURKEYS AND ACEMANNAN AND VERY VIRULENT MAREK'S DISEASE CHALLENGE** | | | | |
|---|---|---|---|---|
| A. Experimental Design | | | | |
| Group | PFU | Quantity of Acm(µg) | Challenge (days) | # of Birds |
| 1 | 0 | 0 | RB1B(5) | 30 |
| 2 | 625 | 0 | RB1B(5) | 29 |
| 3 | 625 | 100 | RB1B(5) | 30 |
| 4 | 1250 | 0 | RB1B(5) | 30 |
| 5 | 1250 | 100 | RB1B(5) | 34 |
| 6 | 3750 | 0 | RB1B(5) | 30 |
| 7 | 3750 | 100 | RB1B(5) | 35 |

| B. Results | | | | |
|---|---|---|---|---|
| Group | # of Dead | # with Tumors | % Affected | P.I. * |
| 1 | 22 | 6 | 90 | 0 |
| 2 | 8 | 10 | 62 | 33 |
| 3 | 1 | 8 | 30 | 69 |
| 4 | 5 | 10 | 50 | 46 |
| 5 | 4 | 9 | 38 | 59 |
| 6 | 3 | 11 | 47 | 49 |
| 7 | 1 | 5 | 17 | 82 |

| | | | | |
|---|---|---|---|---|
| * P.I. = protective index: % of non-vaccinated challenged birds, less the % of vaccinated challenged birds, divided by the % of non-vaccinated challenged birds. Acm = acemannan. | | | | |

### Example 5

### Effect of Acemannan on Early, Virulent Challenge After HVT Vaccination

Day of age birds were divided into groups and administered either zero, 330 or 1000 pfus of the modified live HVT and either zero, one, 100 or 1000 micrograms of acemannan (see Table 5, below). Challenge was at five days post-vaccination, as described in Example 3 (see above).

The results shown in Table 5 suggest that addition of acemannan to HVT vaccine improves the protective quality of the vaccine. Groups receiving acemannan had fewer dead members, a lower percentage of member birds affected by disease and a higher protective index than did corresponding groups not receiving acemannan-containing vaccine.

**Table 5**

| **VACCINATION OF ONE DAY OF AGE CHICKENS WITH MODIFIED LIVE HERPESVIRUS OF TURKEYS AND ACEMANNAN AND VERY VIRULENT MAREK'S DISEASE CHALLENGE** | | | | |
|---|---|---|---|---|
| A. Experimental Design | | | | |
| Group | PFUs | Quantity of Acm(µg) | Challenge (days) | # of Birds |
| 1 | 0 | 0 | RB1B(5) | 21 |
| 2 | 330 | 0 | RB1B(5) | 21 |
| 3 | 330 | 1 | RB1B(5) | 21 |
| 4 | 330 | 100 | RB1B(5) | 21 |
| 5 | 330 | 1000 | RB1B(5) | 21 |
| 6 | 1000 | 0 | RB1B(5) | 21 |
| 7 | 1000 | 1 | RB1B(5) | 21 |
| 8 | 1000 | 100 | RB1B(5) | 20 |
| 9 | 1000 | 1000 | RB1B(5) | 20 |

| B. Results | | | | |
|---|---|---|---|---|
| Group | # of Dead | # with Tumors | % Affected | P.I.* |
| 1 | 16 | 4 | 95 | 0 |
| 2 | 6 | 7 | 62 | 34^{a} |
| 3 | 5 | 7 | 57 | 40 |
| 4 | 4 | 4 | 38 | 60^{b} |
| 5 | 3 | 5 | 38 | 60^{b} |
| 6 | 4 | 1 | 24 | 75 |
| 7 | 4 | 3 | 33 | 65 |
| 8 | 1 | 2 | 15 | 84 |
| 9 | 1 | 4 | 24 | 73 |

| | | | | |
|---|---|---|---|---|
| * P.I. = protective index: % of non-vaccinated challenged birds, less the % of vaccinated challenged birds, divided by the % of non-vaccinated challenged birds. ^{a,b} = Indicates significant differences between vaccine groups as assessed by Fisher's Exact test. Acm = acemannan | | | | |

### Example 6

### Effect of Acemannan on Early, Virulent Challenge After HVT Vaccination

Day of age birds were divided into groups and administered either zero or 360 pfus of the modified live HVT and either zero or 100 micrograms of acemannan (see Table 6, below). Challenge was at five days post-vaccination, as describe in Example 3 (see above).

The data shown in Table 6 suggest that addition of acemannan to HVT vaccine improves the protective capacity of the vaccine. The group receiving vaccine containing acemannan had fewer dead members, a lower percentage of member birds affected by disease and a higher protective index than did the corresponding groups not receiving acemannan-containing vaccine.

**Table 6**

| **VACCINATION OF ONE DAY OF AGE CHICKENS WITH MODIFIED LIVE HERPESVIRUS OF TURKEYS AND ACEMANNAN AND VERY VIRULENT MAREK'S DISEASE CHALLENGE** | | | | |
|---|---|---|---|---|
| A. Experimental Design | | | | |
| Group | PFUs | Quantity of Acm(µg) | Challenge (days) | # of Birds |
| 1 | 0 | 0 | JMV(5) | 20 |
| 2 | 360 | 0 | JMV(5) | 20 |
| 3 | 360 | 0 | JMV(5) | 20 |
| 4 | 360 | 0 | JMV(5) | 20 |
| 5 | 360 | 100 | JMV(5) | 20 |

| B. Results | | | | |
|---|---|---|---|---|
| Group | # of Dead | % Affected | P.I.* | |
| 1 | 19 | 95 | 0 | |
| 2 | 11 | 55 | 42^{a} | |
| 3 | 10 | 50 | 47 | |
| 4 | 10 | 50 | 47 | |
| 5 | 5 | 25 | 74^{b} | |

| | | | | |
|---|---|---|---|---|
| * P.I. = protective index: % of non-vaccinated challenged birds, less the % of vaccinated challenged birds, divided by the % of non-vaccinated challenged birds. ^{a,b} = Indicates significant differences between vaccine groups as assessed by Fisher's Exact test. Acm = acemannan. | | | | |

### Example 7

Table 7 (see below) summarizes the data shown in Examples 3-6. The results from the various tests are batched, and common challenge/vaccine groups are paired. These pairs were assessed for significance by the Wilcoxon matched-pairs signed-rank test. All pairings show that in groups whose members received acemannan-containing vaccine, birds demonstrated improved response to Marek's Disease challenge as compared to birds belonging to groups not receiving acemannan-containing vaccine.

**Table 7**

| **WILCOXON MATCHED-PAIRS SIGNED-RANK TEST; HO: DOES ACEMANNAN AT DOSES OF 100 MICROGRAMS OR ONE MILLIGRAM PER BIRD RESULT IN IMPROVED PERFORMANCE OF HVT WHEN VIEWED INDEPENDENT OF VACCINE DOSE AND CHALLENGE** | | | | | |
|---|---|---|---|---|---|
| Pair | No | Yes | d | Rank of d | Sign |
| 1 | 33 | 69 | 36 | 11 | + |
| 2 | 46 | 59 | 13 | 6 | + |
| 3 | 49 | 82 | 33 | 10 | + |
| 4 | 34 | 60 | 26 | 7.5/8.5 | + |
| 5 | 34 | 60 | 26 | 7.5/8.5 | + |
| 6 | 75 | 73 | 0 | | - |
| 7 | 75 | 84 | 9 | 1.5/2.5 | + |
| 8 | 53 | 77 | 24 | 6/7 | + |
| 9 | 53 | 62 | 9 | 1.5/2.5 | + |
| 10 | 41 | 53 | 12 | 3.5/4.5 | + |
| 11 | 41 | 53 | 12 | 3.5/4.5 | + |
| 12 | 42 | 74 | 32 | 9 | + |
| N = 11/12 | | | | | |
| x N/Pair = 41 | | | | | |
| Sample size = 493 | | | | | |
| P>= 0.01 for a two-tailed test. | | | | | |
| P>= 0.05 for a one-tailed test. | | | | | |

The data suggest that addition of acemannan to HVT vaccine can positively influence the host bird's ability to respond to subsequent challenge. This is particularly significant when an HVT vaccination protocol is stressed by a low plaque forming unit count or an early challenge.

### Example 8

### Preparation of Newcastle's Disease Virus and Infectious Bronchitis Virus Vaccines

Newcastle Disease Virus (NDV) strain B1B1, Chick embryo passage (CEP) 7, fifth passage ("X+5") from the Master Seed, Infectious Bronchitis Virus (IBV) Massachusetts (Mass.) strain, obtained from Intervet, and Herpesvirus of Turkeys FC126 (MDVac) were used in the experiments testing the effect of acemannan upon NDV and IBV vaccination.

Preparation of the spray vaccine is as follows:

| | |
|---|---|
| NDV B1B1, CEP7 (X+5), | IBV Mass. Strain |
| reconstituted to 30 ml diluted 1:1580 | reconstituted to 30 ml. diluted 1:6300 |
| 36 ml. + 36 ml. | |
| 72 ml. total volume Quantity sufficient to 86.4 ml. total volume yields 2.0 log₁₀ EID₅₀ IBV/0.3 ml dose 4.2 log₁₀ TCID₅₀ NDV/0.3 ml dose administered by aerosol spray | |

### Example 9

### Challenge with Newcastle's Disease Virus and Infectious Bronchitis Virus

Newcastle's disease virus strain GB Texas p22 and Infectious bronchitis virus strain M41, obtained from National Veterinary Services Laboratory (NVSL), Ames, Iowa, were used to challenge vaccinated and non-vaccinated birds.

### Example 10

### Effect of Acemannan on a Combination of Newcastle's Disease Virus and Infectious Bronchitis Virus Administered as a Live Day of Age Vaccine

Two hundred day of age chicks were divided evenly into the following groups:

| Group | Treatment | #bds |
|---|---|---|
| 1 | nonvaccinate | 50 |
| 2 | MDVac/NDV+IBV | 50 |
| 3 | ACM/NDV+IBV | 50 |
| 4 | MDVac+ACM/NDV+IBV | 50 |

For Group 2, MDVac was administered as a subcutaneous dose of 0.2 ml. volume, prepared by diluting one ampule of cells in 200 ml of the phosphate buffer diluent described above. For group 3, powdered acemannan (ACM) was suspended in sterile buffer so that a 0.2 ml. subcutaneous injection resulted in a 100 µg/bird dose. In group 4, receiving both Marek's Disease vaccine and acemannan, the Marek's Disease vaccine component was suspended to 2X concentration and mixed 1:1 with a 2X suspension of acemannan in sterile buffer. When 0.2 ml. was administered subcutaneously, it theoretically resulted in the same acemannan dose/bird as Group 3 plus the same MDVac dose/bird as Group 2. NDV and IBV were administered by group as a spray application so that the birds received a theoretical dose of 2.0 log₁₀ EID₅₀ IBV plus 4.2 log₁₀ TCID₅₀ NDV. All vaccinations were given at day-of-age and the birds were then housed separately by group until challenge.

Birds were challenged at 21 days post-vaccination with either the virulent NDV or IBV challenge strain. A total of ten birds per treatment group were deposited into each of the four plexiglass isolators, and two of the isolators were challenged with NDV and two with IBV. This resulted in twenty birds per treatment group challenged intramuscularly with a theoretical dose of 4 log₁₀ TCID₅₀/bird of NDV GB Texas, and twenty birds per treatment group challenged intraocularly of M41 with a theoretical dose of 4 log₁₀ EID₅₀/bird. Ten birds in each treatment group were not vaccinated before challenge. NDV challenge results were recorded as mortality for fourteen days post-challenge. IBV Challenge was assessed by virus reisolation as described in 9 C.F.R. 113.162(c)(3).

Results of NDV challenge are presented in Table 8. Group 4 had the highest percent protection, followed by Group 3. Nonvaccinate controls were significantly affected with no birds surviving the fourteen day post-challenge assessment period.

Results of IBV challenge are also given in Table 8. Again the acemannan-treated groups had a slightly higher percent protection, compared to non-acemannan treated birds. Nonvaccinate controls were significantly (100%) affected.

**Table 8**

| **NDV and IBV Challenge Results** | | | | |
|---|---|---|---|---|
| | NDV CHALLENGE | | IBV CHALLENGE | |
| Group | # Dead/ # Chall. | % Protected | #Dead/ #Chall. | %Protected |
| (1)Nonvaccinate | 20/20 | 0 | 20/20 | 0 |
| (2)MDV/NDV+IBV | 7/20 | 65 | 4/20 | 80 |
| (3)ACM/NDV+IBV | 3/20 | 85 | 0/20 | 100 |
| (4)MDV+ACM/NDV+IBV | 2/20 | 90 | 3/20 | 85 |
| #Chall. = numbered challenged. ACM = acemannan: MDV = MDVac (Marek's Disease vaccine). | | | | |

### Example 11

### Effect of Acemannan on a Combination of Newcastle's Disease Virus and Infectious Bronchitis Virus Administered as a Live Day of Age Vaccine

Approximately 420 day of age chicks were divided into the following groups (Acm = acemannan):

| Group | Treatment | #bds |
|---|---|---|
| 1 | MDVac | 140 |
| 2 | MDVac/NDV+IBV | 140 |
| 3 | MDVac+ACM/NDV+IBV | 140 |

The subcutaneous administration consisted of the same component dose described in Example 10 (see above). The birds were banded for identification purposes, and Group 1 was placed in isolation. The remaining two groups were each divided into eight replicates of 15 birds each and one replicate of 20 birds. For spray vaccination with the NDV + IBV, one replicate from each of the groups was placed into the spray chamber and the vaccine administered using the same component doses per bird as described in Example 10. Birds were resegregated and isolated by group until 21 days post vaccination, when the 20 bird replicates from each group were bled for serum collection. Four of the remaining replicates from each group were challenged with NDV GB Texas and four with IBV M41, as described above. Upon completion of each challenge, the birds were arranged such that one replicate from each of the three original vaccination groups were represented in each of four isolators and challenge for each agent was assessed, as described above.

Results of NDV challenge, are presented by replicate in Table 9 and by group totals in Table 10 (see below). In three of four replicates, the acemannan-treated group (Group 3) demonstrated higher percent protection than the nontreated group (Group 2). In the fourth replicate, the two were equal. The group total percent protection for the acemannan-treated group was 20% higher than the untreated group. All nonvaccinate control birds (Group 1) died during the 14 day observation period.

Results of IBV challenge are also given in Tables 9 and 10. Only two of the four replicates were tested. For one set of replicates, the acemannan-treated group outperformed its untreated counterpart, but for the other set of replicates it did not. Of the 27 nonvaccinate control birds tested, only one was not affected.

**Table 9**

| **NDV and IBV Challenge Results as Presented by Replicate** | | | | |
|---|---|---|---|---|
| | NDV CHALLENGE | | IBV CHALLENGE | |
| Group | # Dead/ # Chall. | % Protected | # Dead/ #Chall. | %Protected |
| Replicate 1 | | | | |
| (1)MDV | 15/15 | 0 | 13/14 | 7 |
| (2)MDV/NDV+IBV | 4/15 | 73 | 3/13 | 77 |
| (3)MDV+ACM/NDV+IBV | 0/15 | 100 | 3/15 | 80 |

| Replicate 2 | | | | |
|---|---|---|---|---|
| (1)MDV | 15/15 | 0 | 13/13 | 0 |
| (2)MDV/NDV+IBV | 5/15 | 67 | 3/15 | 80 |
| (3)MDV+ACM/NDV+IBV | 1/15 | 93 | 4/14 | 71 |

| Replicate 3 | | | | |
|---|---|---|---|---|
| (1)MDV | 15/15 | 0 | n.t. | n.t. |
| (2)MDV/NDV+IBV | 1/15 | 93 | n.t. | n.t. |
| (3)MDV+ACM/NDV+IBV | 1/15 | 93 | n.t. | n.t. |

| Replicate 4 | | | | |
|---|---|---|---|---|
| (1) MDV | 15/15 | 0 | n.t. | n.t. |
| (2)MDV/NDV+IBV | 4/15 | 73 | n.t. | n.t. |
| (3)MDV+ACM/NDV+IBV | 3/15 | 80 | n.t. | n.t. |
| n.t. = Not tested. ACM = acemannan; MDV = MDVac (Marek's Disease vaccine). | | | | |

**Table 10**

| **NDV and IBV Challenge Results as Presented By Group Totals** | | |
|---|---|---|
| A. NDV CHALLENGE | | |
| Group | # Dead/ # Chall. | **%** Protected |
| (1)MDV | 60/60 | 0 |
| (2)MDV/NDV+IBV | 14/60 | 77 |
| (3)MDV+ACM/NDV+IBV | 5/50 | 92 |

| B. IBV CHALLENGE | | |
|---|---|---|
| Group | #Dead/ #Chall. | % Protected |
| (1)MDV | 26/27 | 4 |
| (2)MDV/NDV+IBV | 6/28 | 79 |
| (3)MDV+ACM/NDV+IBV | 7/29 | 76 |
| #Chall. = number challenged. ACM = acemannan; MDV = MDVac (Marek's Disease vaccine). | | |

Results of serology are given in Table 11 (see below). For NDV, 57% of the acemannan-treated birds tested had an ELISA profile rank greater than 4, whereas in the untreated vaccinates only half that many (23.5%) had an ELISA profile rank greater than 4. For IBV, very little serologic response was seen in either group.

**Table 11**

| **NDV and IBV Serology Results** | | | | | |
|---|---|---|---|---|---|
| | NDV ELISA | | | IBV ELISA | |
| Group | mean NDV profile | # birds profile>4 (%) | | mean IBV profile | # birds profile >4 (%) |
| (1) MDV | 0 | 0 | (0) | 0 | 0 (0) |
| (2) MDV/ NDV+IBV | 1.88 | 4/17 | (23.5) | 0.82 | 2/17(11.7) |
| (3) MDV+ACM/ NDV+IBV | 3.71 | 8/14 | (57.1) | 0.93 | 1/14 (7.1) |
| Acm = acemannan. | | | | | |

### Example 12

NDV challenge results from both sets of experiments involving NDV vaccination indicate that there was an increase in protection in the acemannan-treated groups. The only instance in which this increase was not observed was in replicate 4 of Example 11 where both vaccinate groups had equivalent protection. There is inherent variability in the efficiency of vaccination when using the spray cabinet. For this reason, we vaccinated each replicate group on separate occasions, so that each acemannan-treated or non-treated comparison would receive identical NDV/IBV vaccine exposure. The trend we see in the replicates is that as the vaccination becomes more effective (see Group 3), the differences between acemannan-treated and non-treated groups becomes less.

IBV challenge results were variable. In Example 10, there appeared to be an increase in protection in the acemannan-treated groups, while this was not the case in Example 11. It may be that there is more variability inherent to assessment of challenge protection by virus reisolation than by mortality.

ELISA serology results in Example 11 suggest that there was an increased antibody response to the NDV fraction of the spray vaccine when the birds were treated with acemannan, possibly contributing to the observed increase in protection. The ELISA results for IBV were too low in general to be of any use.

Treatment with acemannan at day of age elicited increased serologic and protective responses to the NDV B1B1 component of the spray administered combination vaccine compared to the non-acemannan treated control group.

Treatment with acemannan at day of age had little or no effect on the serologic or protective responses induced by IBV Mass. component of the spray administered vaccine.

### Example 13

### Interference Testing of Bursine 2 and Cell-Associated HVT Vaccines in Day Old SPF Chickens. With or Without the Addition of Acemannan

### Preparation of Infectious Bursal Disease Virus Vaccine

Infectious bursal disease virus (IBDV) Bursine 2, release titer 10^{4.7} TCID₅₀ per bird dose, and Herpesvirus of turkeys FC126, release titer 9,415 plaque forming units per bird dose in phosphate buffer diluent (described above) were used. Bursine 2 was obtained from Phil Lukert, University of Georgia.

The resuspended HVT vaccine was held at refrigerated temperatures for two hours prior to titration. Plaque forming unit titrations were performed on secondary chicken embryo fibroblast (CEF) monolayers. Infectious bursal disease virus was titered on primary CEF.

### Vaccination stock materials were prepared as follows:

**Stock A.** One vial of HVT was thawed and diluted in 100 milliliters (ml) of phosphate buffer.

**Stock B.** One vial of Bursine 2 Serial was reconstituted to 50 ml using phosphate buffer.

**Stock C.** A 2.0 milligram per ml solution of acemannan was prepared using phosphate buffer to rehydrate the acemannan powder.

**Stock D.** Twenty-five ml of Stock B plus 25 ml of phosphate buffer.

**Stock E.** Twenty-five ml of Stock B and 25 ml of Stock C.

One-day-old SPF leghorn chicks were vaccinated subcutaneously in the nape of the neck with combinations of the stock solutions as listed below. All vaccinations were 0.2 ml in volume.

**Vaccination Group 1.HVT only.** Twenty ml of Stock A was mixed with an additional 20 ml of the phosphate buffer prior to injection and 0.2 ml doses were then administered to twenty chicks.

**Vaccine Group 2. Bursine 2 and HVT.** Twenty ml of Stock A was mixed with 20 ml of Stock D and 0.2 ml doses were then administered to forty chicks.

**Vaccine Group 3. Bursine 2 only.** Twenty ml of Stock D was mixed with an additional 20 ml of the phosphate buffer before injecting a group of twenty chicks with 0.2 ml doses.

**Vaccine Group 4. Bursine 2-Acemannan.** Twenty ml of Stock E was combined with an additional 20 ml of the phosphate buffer and 0.2 ml doses were then administered to twenty chicks.

**Vaccine Group 5. Bursine 2-HVT-Acemannan.** Twenty ml of Stock A and 20 ml of Stock E were combined and were then administered to forty chicks. ,

The birds used in these studies were SPF white leghorns vaccinated at one day of age. Forty chicks from the same hatch were held in plexiglass isolation units for use as challenge controls.

Bursal Disease challenge virus was supplied by National Veterinary Services Laboratory, Ames, Iowa. The virus was diluted 1:5 in tryptose phosphate broth to yield a 10^{1.4}EID₅₀ eyedrop bird dose. The Marek's Disease challenge virus was strain RB1B/17 T55, passage CP1, TCP3, CP1, TCP4. The material had a titer of 2.8x 10^{4.0} plaque forming units (pfus) per 0.2 milliliters. The virus was diluted in phosphate buffer to contain 1000 pfus per bird challenge dose.

Five days post vaccination 20 controls. Vaccine Group 1, and 20 birds each from Vaccine Group 2 and 5 were challenged intraperitoneally (IP) with the RB1B isolate of Marek's Disease Virus. Fourteen days after vaccination, ten birds were bled from Vaccine Groups 2,3,4,5 and the non-challenged controls for the determination of IBDV beta method serum neutralization (SN) titers. Twenty chickens each from Groups 2,3,4,5 and the controls were subsequently challenged by eyedrop with the NVSL IBDV virus. Birds challenged with IBDV were sacrificed eleven days later, weighed, and their bursas removed and weighed to establish a bursa to body weight ratio as an evaluation of clinical bursa atrophy. All MDV challenged birds that died up to day 48 of the study were necropsied and scored for clinical lesions. The survivors were sacrificed on day 48 and also scored for lesions.

The circulating antibody titers to IBDV were evaluated by SN testing.

Marek's Disease immunity is reported as a protective index (PI) where the performance of each group is compared to the infectivity level of the control. Protective Index = (((% positive controls) - (% positive vaccinates))/(% positive controls)) x 100.

Bursal disease protection is reported as the percentage of immune birds based on atrophy of the bursa. This is judged using a bursal index (BI = (bursa weight/body weight) x 100). A value greater than or equal to 2.5 is considered protected.

The results of post mortem clinical lesion evaluation for Marek's Disease are listed in Table 12 (see below) as protective indices. The control group, which has a PI of zero by definition, was 94 percent positive for clinical lesions. Birds administered HVT, HVT plus Bursine 2, or the HVT-Bursine 2-Acemannan combination were 83, 89, and 95 percent free of clinical lesions, respectively. These figures extrapolate to protective indices of 82, 88, and 95.

**Table 12**

| **Marek's Disease Protective Indices** | |
|---|---|
| Treatment | Protective Index |
| CA HVT | 82 |
| CA HVT-Bursine 2 | 88 |
| CA HVT-Bursine 2-Acemannan | 95 |
| Controls | 0 |
| CA = cell associated. | |

The bursal disease results are shown in Table 13 as the bursa to body weight ratios with the percentage of birds clinically immune in each group noted in parentheses. For each vaccine tested there was a numerical increase in protection with the addition of acemannan. Birds given Bursine 2 alone were 85 percent protected compared to 100 percent immunity with the addition of 100 micrograms acemannan per bird dose. With the Bursine 2-HVT combination, the group with acemannan addition held a 100 percent to 79 percent clinical protection advantage.

**Table 13**

| **Bursal Disease Bursa-To-Body Weight Ratios** | | |
|---|---|---|
| Treatment | B/BW Ratio + S.D. | Percent Immune |
| Bursine 2 | 3.9±1.5 | 85 |
| Bursine 2-Acemannan | 4.9±1.6 | 100 |
| Bursine 2-HVT | 4.0±1.6 | 79 |
| B2-HVT-Acemannan | 5.0±1.5 | 100 |
| Controls | 1.1±0.3 | 0 |
| B/BW = Bursa/body weight; B2 = Bursine 2. | | |

Bursal Index (bursa to body weight ratio) average values ± one standard deviation of the mean for each vaccinate group were 3.9±1.5, 4.9±1.6, 4.0±1.6, and 5.0±1.5 for the Bursine 2, Bursine 2-Acemannan, Bursine 2-HVT, and Bursine 2-HVT-Acemannan groups, respectively. Independent sample Student's T-test analysis of the individual bursa-to-body weight ratios (see Table 14, below) demonstrates that the groups representing only Bursine 2 are not significantly different, yet the Bursine 2-HVT comparison shows that the acemannan group was significantly better at the 95 percent confidence level.

**Table 14**

| **Individual Bursa-to Body Weight Ratios** | | | | |
|---|---|---|---|---|
| Bursine 2 | Bursine 2+ Acemannan | Bursine 2^{a}+ HVT | B-2+HVT^{a}+ Acemannan | C |
| 1.7 | 5.1 | 1.7 | 7.0 | 1.3 |
| 5.1 | 4.3 | 4.5 | 7.4 | 1.4 |
| 3.1 | 5.2 | 1.4 | 5.6 | 0.7 |
| 2.9 | 5.5 | 2.2 | 6.0 | 1.6 |
| 3.1 | 4.3 | 5.5 | 8.2 | 0.5 |
| 3.1 | 4.0 | 3.5 | 7.3 | 1.2 |
| 1.2 | 5.0 | 3.2 | 4.5 | 1.0 |
| 3.0 | 3.9 | 5.3 | 4.3 | 1.3 |
| 4.5 | 4.0 | 4.6 | 4.6 | 0.8 |
| 4.7 | 4.8 | 5.6 | 4.7 | 1.4 |
| 2.0 | 3.6 | 5.1 | 3.2 | 1.1 |
| 5.3 | 8.4 | 2.9 | 4.5 | 0.6 |
| 3.6 | 6.2 | 5.0 | 5.2 | 1.4 |
| 4.7 | 9.0 | 5.2 | 3.3 | 1.1 |
| 6.9 | 3.5 | 6.5 | 3.3 | 1.3 |
| 5.7 | 2.9 | 2.8 | 3.7 | 1.3 |
| 2.7 | 4.4 | 3.8 | 4.4 | 1.0 |
| 4.1 | | 6.1 | 4.3 | 1.2 |
| 4.3 | | 1.7 | 4.1 | 1.0 |
| 6.3 | | | 5.3 | |

| Average ± SD | | | | |
|---|---|---|---|---|
| 3.9± | 4.9± | 4.0± | 5.0± | 1.1± |
| 1.5 | 1.6 | 1.6 | 1.5 | 0.3 |

| | | | | |
|---|---|---|---|---|
| ^{a}These groups are significantly different; T = 2.075998. C = Controls. | | | | |

Serology results are shown in Table 15 (see below). The SN titers did not follow any correlative pattern with the protection from clinical disease. Bursal SN titers were greatest in the Bursine 2-HVT-Acemannan (GMT of 69), and lowest in the birds given the combination without acemannan (GMT of 30). For chickens given the monovalent IBDV vaccine, the group without acemannan had a GMT of 56 as compared to 35 with the additive. Controls were all ≥2.

**Table 15**

| **Bursal Serology** | |
|---|---|
| Treatment | SN^{b} |
| Bursine 2 | 56 |
| Bursine 2-ACM | 35 |
| Bursine 2-HVT | 30 |
| Bursine 2-HVT-ACM | 69 |
| Controls | ≤2 |

| | |
|---|---|
| a= Arithmetic mean profile rank. b= Geometric mean neutralization titer. | |

No interference in immunity was observed between Bursine 2 and cell associated HVT vaccines when both were administered at a full field dose.

The addition of acemannan to the monovalent Bursine 2 vaccine and the combination with HVT resulted in a numerical efficacy advantage for the monovalent treatment containing acemannan, and for the HVT in the combination vaccine. The Bursine 2 in the combination was significantly better with acemannan (95 percent confidence) than without.

### Example 15

### Piglet Vaccination/Challenge Study With and Without Acemannan

One hundred seventy (170) suckling one-week-old piglets were purchased from Jensen Brothers Inc., Thornton, IA. All were housed on slatted floor pens in three rooms containing not more than twenty piglets per pen.

One hundred twenty (120) CF1 16-22 gram white mice were purchased from Harlan Sprague Dawley. All mice were housed in groups of eight mice per box.

There were ten animals per group but because of the number of pigs needed to complete the study, replicate sets of 5 pigs each were selected from farrowing one week apart. within a replicate, pigs were randomly assigned to a vaccination or control group. Vaccines were mixed with and without acemannan at 5 mg/dose. Additionally, acemannan was evaluated as an immunostimulant prior to vaccination in a group of 10 pigs which were selected when 1 day old. The different vaccine and control groups are listed in Table 1. Pigs were vaccinated at one week of age and again at 3 weeks of age. The first vaccination was a 2.0 ml dose of bacterin given intramuscularly in the neck between the shoulder and the ear. The second vaccination was similarly given on the opposite side of the neck.

**Table 16**

| **EXPERIMENTAL GROUPS AND VACCINE COMPOSITION**^{**a**} | | |
|---|---|---|
| Vaccine Group | PMD^{b} Concentration | Acemannan^{c} |
| PMD-1 | 8.7 | None |
| PMD-2 | 9.0 | None |
| PMD-3 | 9.5 | None |
| 3-Way+D-1^{d} | 8.7 | None |
| 3-Way+D-2 | 9.0 | None |
| 3-Way+D-3 | 9.5 | None |
| PMD-1A | 8.7 | 5 mg |
| PMD-2A | 9.0 | 5 mg |
| PMD-3A | 9.5 | 5 mg |
| 3-Way+D-1A | 8.7 | 5 mg |
| 3-Way+D-2A | 9.0 | 5 mg |
| 3-Way+D-3A | 9.5 | 5 mg |
| 3-Way+D+Tox^{e} | 9.0 | None |
| 3-Way | None | None |
| Neonatal Acemannan^{f} | None | 5 mg, 3X |
| (3-Way+D-2) | 9.0 | None |
| Non-vaccinated Challenges^{g} | None | None |
| Non-vaccinated Non-Challenged^{h} | None | None |

| | | |
|---|---|---|
| ^{a} All bacterins contained the following components: a. Aluminum hydroxide (Alhydrogel '85') 0.75% (as Al₂O₃) b. Formaldehyde 0 . 2 % residual c. Saline Q.S. to 2.0 ml dose | | |
| ^{b} PMD = Pasteurella multocida, Type D. Values listed are Log₁₀ of the concentration (CFU) per dose. | | |
| ^{c} Acemannan is an acetylated mannose polymer extracted from Aloe vera plants. It was supplied and added to vaccines as a desiccated powder to a final amount of 5 mg per dose. | | |
| ^{d} In addition to components already listed, 3-Way bacterins also contained: a. Bordetella bronchiseptica 3.0 x 10⁹ CFU/dose b. Erysipelothrix rhusiopathiae 1.0 ml/dose (The E. rhusiopathiae had a concentration factor of 13.1 over raw culture) c. Pasteurella multocida, Type A 3.0 x 10⁹ CFU/dose. | | |
| ^{e} Inactivated, Concentrated Broth Supernate of the PMD culture was added to give the amount of toxoid present in broth of the PMD-2 bacterin (in addition to any toxoid activity already present in the standard formulation). | | |
| ^{f} One litter of 10 pigs was given a saline suspension of Acemannan (5 mgs/dose - 1 ml) at 1, 3, and 5 days of age. The 3-Way+2-D bacterin was given at 1 and 3 weeks of age as for other pigs in replicate 2. | | |
| ^{g} Non-vaccinates were bled as other pigs, but were given no other infections. Challenged pigs were mixed with vaccinated challenged pigs. | | |
| ^{h} Non-vaccinate, non-challenge pigs were kept isolated from other pigs after challenge of the other pigs. No mock challenge was given. | | |

One week after the second vaccination, pigs were sedated with a 1:10 PromAce:Vetalar [Ketamine] mixture, about 1.0 ml/10 1b body weight. When the pigs were sedate, 2 ml/nostral of the challenge suspension was administered via intranasal intubation. Pigs were intermittently observed until recovery from sedation.

Two hundred milliliters of an X+6 passage of P. multocida Type D (PMD), strain P1683-T3, was inoculated into ten liters of Difco Bitek Fermentation Broth (BT). The culture was fermented to an optical density (OD₆₆₀ₙₘ) of 1.45 which contained 4.3 x 10⁹ CFU/ml. The culture was inactivated by adding 20.0 ml formaldehyde, stirring at 37°C for one hour, then at 4°C for five days. The inactivated culture was concentrated 5.3 fold with an Amicon DC-10 100,000 dalton (molecular weight) cut-off hollow fiber filter. Compositions of the bacterins is presented in Table 16 (see above). Briefly, in multi-component vaccines without acemannan, the PMD concentration was varied at 5, 10, and 30 x 10⁸ CFU/dose while the other components were held constant. The same concentrations of PMD were used in the single component 'reference' vaccines. Additionally, similar groups of vaccines were prepared with acemannan at 5 mg/dose.

Inactivation was assessed by incubating one ml of each of the fourteen bacterin in both thioglycollate and tryptic soy broth at 25°C and 37°C for fourteen days before being considered inactive.

Safety of the bacterins was assessed by inoculating eight mice subcutaneously with 0.5 ml. of a given bacterin formulation. Mice were evaluated for adverse effects attributable to the injection.

An X passage of PMD strain P1683-T3, was inoculated into two flasks containing 75.0 ml of BT broth. The cultures were incubated at 37°C for 4.5 hours while being shaken at 125 revolutions per minute. At an OD₆₆₀ₙₘ of 1.65, the culture was diluted 1:4 in BT broth and chilled on ice until used. A viable cell count showed that the challenge contained 4.5 x 10⁹ and 5.6 x 10⁹ CFU for the two replicates.

Piglets were bled from the anterior vena cava at the following times: first vaccination (V1), second vaccination (V2), challenge (C+0) and necropsy (C+21). In all cases, blood was collected before any bacterin, sedative, challenge, or euthanasia solution was administered. Blood samples were centrifuged at 250C x g for 20 minutes at 4°C. Serum was collected, heat inactivated for 30 minutes at 56°C, and stored at -20°C until tested.

Enzyme linked immunosorbant assays (ELISA) were performed on all serum samples to evaluate the antibody response to the antigenic components of the bacterins and to help detect interference between components.

Pigs were observed for adverse effects of vaccination and subsequently for clinical signs attributable to challenge. Because of the number of vaccine groups and pigs per pen, specific signs for a given pig were not recorded. Pigs that died during the 3 week observation period were necropsied and evaluated for specificity of death. Pigs dying of causes not attributable to challenge were excluded from further evaluation. Surviving pigs were euthanized with an overdose of sodium pentabarbitol and necropsied three weeks after challenge.

At necropsy, lung lesions and vaccination site reactions were noted and scored. The snouts were removed nostril to the eye sockets, labelled and later sawed on a band saw into one-half inch sections at the level of the second premolar. Sections were photographed alongside of millimeter scale and subsequently 5 x 7 inch color photographs were printed from the negatives.

All scoring systems are presented in Table 17 (see below). Severity of turbinate atrophy was evaluated morphometrically from the 5 X 7 prints. The morphometry protocol is that described by Collins, et al. (Turbinate perimeter ratio as an indicator of conchal atrophy for diagnosis of atrophic rhinitis in pigs. Am. J. Vet. Res., 50(3), 1989: 421-424. Average turbinate area ratios (TAR) and average turbinate perimeter ratios (TPR) of treatment groups were analyzed for significant differences by analysis of variance.

**Table 17**

| **SCORING SYSTEMS** | | |
|---|---|---|
| A. Lung Scoring System: Scoring All Lobes (4pts/lobe; 24 pts total) | | |
| 1. | Partial consolidation of lobe | (+1) |
| 2. | Whole consolidation of lobe | (+1) |
| 3. | Abscess | (+1) |
| 4. | Fibrinous pleuritis | (+1) |

| B. Vaccination Site Reaction: (Two Sites) | | |
|---|---|---|
| 1. | Nothing to mild fibrinous streaks - 0 points | |
| 2. | Small (< 1 cm) - 1 point | |
| 3. | Up to 1 cm x 3 cm - 2 points | |
| 4. | > 1 cm x 3 cm - 3 points | |
| 5. | Large abscess with pus - 4 points | |

All bacterins prepared were safe as indicated by inoculation of mice as per 9 C.F.R. guidelines for safety. Bacterins that contained acemannan were contaminated with a mixed culture of organisms. However, the vaccines appeared to be inactive with respect to the vaccine strains.

Tables 18 and 19 (see below) summarize the clinical and gross pathologic results of challenge for the different groups. Lung lesions were mild in all groups. Likewise, incidence of mortality was low. Vaccination site reactions were generally mild, but the most severe reactions tended to occur in vaccines with the greatest biomass. Among those, if acemannan was present, the lesions were most severe. Some abscesses resulting from injection with vaccines containing acemannan were 5 to 10 cm in diameter.

**Table 18**

| **MORTALITY AND GROSS NECROPSY LESIONS RESULTING FROM VACCINATION AND CHALLENGE WITH PASTEURELLA MULTOCIDA, TYPE D** | | | |
|---|---|---|---|
| Vaccine Group^{a} | Lung Score^{b} | Vaccination Reactions ^{c} | Mortality^{d} |
| PMD-1 | 0.00 | 0.44/0.00 | 1/9 |
| PMD-2 | 0.00 | 0.00/0.22 | 0/10 |
| PMD-3 | 1.20 | 0.70/0.80 | 0/10 |
| 3-Way+D-1 | 0.50 | 1.20/1.00 | 0/10 |
| 3-Way+D-2 | 0.00 | 0.56/1.33 | 1/10 |
| 3-Way+D-3 | 0.00 | 1.75/2.00 | 2/10 |
| 3-Way+D+Tox | 0.22 | 0.22/1.00 | 1/10 |
| 3-Way | 2.67 | 1.00/1.11 | 1/10 |
| Non-Vacc. Challenged | 0.56 | ----/---- | 2/11 |
| Non-Vacc. Non-Challenged | 0.11 | ----/---- | 1/10 |

| | | | |
|---|---|---|---|
| ^{a} Vaccine groups are as listed in Table 16. The two replicate groups have been combined. Acemannan groups are in Table 19 (see below). | | | |
| ^{b} Method of evaluating lung lesions are in Table 17. The maximum score is 24.0. Values are the mean for the number of pigs in the group. | | | |
| ^{c} Method of evaluating vaccine reactions is in Table 17. The maximum score is 4.0 per site. The values are the means for 1st/2nd vaccination site. | | | |
| ^{d} Ratios represent number dead after challenge over the number in the group. | | | |

**Table 19**

| **MORTALITY AND GROSS NECROPSY LESIONS RESULTING FROM VACCINATION AND CHALLENGE WITH PASTEURELLA MULTOCIDA, TYPE D; ACEMANNAN WAS IN THE VACCINES AT 5 MG PER DOSE** | | | |
|---|---|---|---|
| Vaccine Group^{a} | Lung Score^{b} | Vaccination Reactions ^{c} | Mortal ity^{d} |
| PMD-1A | 0.00 | 0.13/0.00 | 0/10 |
| PMD-2A | 1.30 | 0.30/0.20 | 2/10 |
| PMD-3A | 1.33 | 0.22/0.89 | 1/10 |
| 3-Way+D-1A | 1.22 | 0.33/0.11 | 1/10 |
| 3-Way+D-2A | 0.40 | 1.40/0.40 | 0/10 |
| 3-Way+D-3A | 1.63 | 2.38/2.63 | 2/10 |
| Neonatal Acemannan^{e} 3-Way+D-2 | 0.00 | 1.44/1.67 | 1/10 |
| Non-Vacc. Challenge | 0.56 | ----/---- | 2/11 |
| Non-Vacc. Non-Chall. | 0.11 | ----/---- | 1/10 |

| | | | |
|---|---|---|---|
| ^{a} Vaccine groups are as listed in Table 16. The two replicate groups have been combined; Non-Vacc. = nonvaccinated. | | | |
| ^{b} Method of evaluating lung lesions are in Table 17. The maximum score is 24.0 per site. Values are the mean for the number of pigs in the group. | | | |
| ^{c} Method of evaluating vaccine reaction is in Table 17. The maximum score is 4.0 per site. The values are the means for 1st/2nd vaccination site. | | | |
| ^{d} Ratios represent number dead after challenge over the number in the group. | | | |
| ^{e} Pigs were given injections of an Acemannan suspension (5mg per ml) when 1, 3, and 5 days old. When seven and 21 days old, pigs were vaccinated with the 3-Way+D-2 (no additional Acemannan at that time). | | | |

Average turbinate perimeter ratios (TPR) and turbinate area ratios (TAR) are presented in Tables 20 and 21 (see below). The TPR for non-vaccinated, non-challenged pigs was significantly (P ≥ 0.05) greater than all other groups except the Neonatal acemannan group (P = 0.1) in a one-tailed T-test. Three vaccines (Groups PMD-1, PMD-2, and 3-Way+D-2) conferred significant protection against challenge with the live, toxigenic PMD. Note that both PMD-2A and 3-Way+D-2A also conferred significant protection, but presence of acemannan in those products did not appear to confer any added benefit with respect to TPR. The TAR of the Non-vaccinated, Non-challenged pigs was significantly greater than that of all other groups. Only the PMD-1 and PMD-1A vaccines conferred significant protection as detected by TAR against the PMD challenge. Again, the addition of acemannan did not appear to be beneficial with respect to TAR.

**Table 20**

| **MORPHOMETRIC EVALUATION OF TURBINATE LESIONS IN THE COMBINED REPLICATES** | | | |
|---|---|---|---|
| Vaccine Group^{a} | No. of Pigs^{b} | TAR Mean+(S.D.) | TPR Mean+(S.D.) |
| PMD-1 | 9 | 0.426 (0.083)^{c} | 1.096 (0.268)^{c} |
| PMD-2 | 9 | 0.396 (0.107) | 1.109 (0.201)^{c} |
| PMD-3 | 9 | 0.319 (0.094) | 0.857 (0.292) |
| 3-Way+D-1 | 10 | 0.403 (0.108) | 1.004 (0.292) |
| 3-Way+D-2 | 9 | 0.409 (0.109) | 1.056 (0.245)^{c} |
| 3-Way+D-3 | 8 | 0.359 (0.072) | 0.903 (0.232) |
| 3-Way+D+Tox | 9 | 0.379 (0.087) | 0.951 (0.177) |
| 3-Way | 9 | 0.367 (0.084) | 1.000 (0.183) |
| Non-Vacc. Challenge | 9 | 0.347 (0.099) | 0.856 (0.282) |
| Non-Vacc. Non-Challenge | 9 | 0.555 (0.100)^{d} | 1.369(0.167)^{d} |

| | | | |
|---|---|---|---|
| Values are the average + (standard deviation) of turbinate area (TAR) and turbinate perimeter (TPR) ratios as evaluated by digitized tracings of turbinate and nasal cavity from 5 x 7 photographs of snout sections through the 2nd premolar. ^{a} Vaccine groups are as listed in Table 1. The two replicate groups have been combined. | | | |
| ^{b} Number of pigs surviving to 3 weeks after challenge. | | | |
| ^{c} These values are significantly better (P≤0.05; 1-tailed T-test) than the value in the same column for the Non-vaccinated challenged pigs. | | | |
| ^{d} These values are significantly better than any other values in the same column. | | | |

**Table 21**

| **MORPHOMETRIC EVALUATION OF TURBINATE LESIONS IN THE COMBINED REPLICATES OF PIGS VACCINATED WITH VACCINES CONTAINING ACEMANNAN** | | | |
|---|---|---|---|
| Vaccine Group^{a} | No. of Pigs^{b} | TAR Mean +/- (S.D.) | TPR Mean +/- (S.D.) |
| PMD-1A | 8 | 0.412 (0.038)c | 1.075 (0.141)^{c} |
| PMD-2A | 10 | 0.346 (0.107) | 1.011 (0.301) |
| PMD-3A | 9 | 0.254 (0.033) | 0.727 (0.126) |
| 3-Way+D-1A | 9 | 0.394 (0.074) | 1.002 (0.281) |
| 3-Way+D-2A | 10 | 0.410 (0.099) | 1.112 (0.233) |
| 3-Way+D-3A | 8 | 0.270 (0.070) | 0.566 (0.288) |
| Neonatal Acemannan 3-Way+D-2 | 9 | 0.413 (0.095) | 1.204 (0.215)^{c}^{d} |
| Non-Vacc. Challenge | 9 | 0.347 (0.099) | 0.856 (0.282) |
| Non-Vacc. Non-Chall. | 9 | 0.555 (0.100)^{c} | 1.369 (0.167)^{e} |

| | | | |
|---|---|---|---|
| Values are the average +/- (standard deviation) of turbinate area (TAR) and turbinate perimeter (TPR) ratios as evaluated by digitized tracings of turbinate and nasal cavity form 5 x 7 photographs of snout sections through the 2nd premolar. ^{a} Vaccine groups as listed in Table 16. The two replicate groups have been combined. Acemannan, when present, was at a concentration of 5 mg per dose. The neonatal acemannan group was given 5 mg in suspension at 1, 3, and 5 days of age followed by vaccination with the 3-way+D-2 vaccine at 7 and 21 days of age. | | | |
| ^{b} Number of pigs surviving to 30 weeks after challenge. | | | |
| ^{c} These values are significantly better (P≤ 0.05; 1-tailed T-test) than the value in the same column for the non-vaccinated, challenged pigs. | | | |
| ^{d} This value is not significantly different from that for the non-vaccinated, non-challenged groups. | | | |
| ^{e} These values are significantly better than any others, except as noted in footnote "d", in the same column. | | | |

ELISA tests were run to detect IgG antibody response to each of the component organisms in the full combination bacterin on all fractions of the bacterin-toxoids. The data presented in Table 22 (see below) indicates no interference by the B. bronchiseptica, P, multocida, and E. rhusiopathiae components on the response to PMD in the combination bacterin. Although the lower two doses of the monovalent PMD vaccine conferred significant protection against atrophic rhinitis, the antibody response to these two vaccines was weak, even after challenge in the case of the PMD-1 vaccine. The antibody response of the three combination bacterins was consistently higher than any other vaccine noted in Table 22 (see below).

**Table 22**

| **SEROLOGIC RESPONSE (IGG) TO PASTEURELLA MULTOCIDA, TYPE D (PMD), IN PIGS VACCINATED WITH BACTERINS CONTAINING PMD** | | | | |
|---|---|---|---|---|
| Vaccine Group^{a} | V1 | V2 | C+0 | C+21 |
| PMD-1 | 0.892 | 0.727 | 0.751 | 0.701 |
| PMD-2 | 0.969 | 0.806 | 0.744 | 0.920 |
| PMD-3 | 0.818 | 0.758 | 0.969 | 1.088 |
| 3-Way+D-1 | 0.977 | 0.717 | 1.305 | 1.143 |
| 3-Way+D-2 | 0.845 | 0.721 | 1.039 | 1.022 |
| 3-Way+D-3 | 0.669 | 0.819 | 1.029 | 1.117 |
| 3-Way+D+Tox | 1.072 | 0.762 | 0.934 | 0.927 |
| 3-Way | 0.906 | 0.776 | 0.894 | 1.010 |
| Non-Vacc. Challenge | 0.835 | 0.612 | 0.697 | 1.025 |
| Non-Vacc. Non-Chall. | 0.800 | 0.614 | 0.791 | 0.914 |

| | | | | |
|---|---|---|---|---|
| Comparison was made to bacterins without PMD and to serum from non-vaccinated pigs to evaluate interference between other component organisms and the PMD. serums were collected at the time of first vaccination (V1), time of second vaccination (V2), time of challenge (C+0) and time of Necropsy (C+21). Response was measured by ELISA tests with a PMD whole-cell sonicate antigen coated onto the test wells. ^{a} Vaccine groups are as listed in Table 16. The two replicate groups have been combined. | | | | |

As Table 23 (see below) indicates, there may have been interference by PMD with PMA at the two higher concentrations of the PMD in the combination bacterins. However, this may be anomalous since the response to PMA in the 3-Way+D+Tox vaccine which has the same concentration of PMD as the 3-Way+D-2 was similar to the response to the 3-Way vaccine. The response to Bordetella and Erysipelothrix was good in all vaccines with no apparent interference.

**Table 23**

| **SEROLOGIC RESPONSE (IGG) TO PASTEURELLA MULTOCIDA, TYPE A (PMA), BORDETELLA BRONCHISEPTICA (BB), AND ERYSIPELOTHRIX RHUSIOPATHIAE (E. RHU), IN PIGS VACCINATED WITH BACTERINS CONTAINING THOSE COMPONENTS** | | | | |
|---|---|---|---|---|
| Vaccine Group^{a} | V1 | V2 | C+0 | C+21 |
| PMD-1 | | | | |
| PMA | 0.675 | 0.669 | 1.245 | 1.277 |
| BB | 0.982 | 0.650 | 1.353 | 1.684 |
| E. rhu | 1.012 | 0.847 | 1.317 | 1.270 |
| | | | | |

| 3-Way+D-1 | | | | |
|---|---|---|---|---|
| PMA | 0.581 | 0.756 | 1.171 | 1.067 |
| BB | 0.899 | 0.730 | 1.282 | 1.766 |
| E. rhu | 0.887 | 0.774 | 1.262 | 1.274 |
| | | | | |

| 3-Way+D-2 | | | | |
|---|---|---|---|---|
| PMA | 0.556 | 0.650 | 0.665 | 1.123 |
| BB | 0.667 | 0.626 | 1.362 | 1.686 |
| E. rhu | 0.860 | 0.883 | 1.327 | 1.317 |
| | | | | |

| 3-Way+D-3 | | | | |
|---|---|---|---|---|
| PMA | 0.439 | 0.668 | 0.634 | 1.194 |
| BB | 0.723 | 0.785 | 1.576 | 1.794 |
| E. rhu | 0.991 | 0.651 | 1.255 | 1.218 |
| | | | | |

| 3-Way+D+Tox | | | | |
|---|---|---|---|---|
| PMA | 0.675 | 0.758 | 1.179 | 0.904 |
| BB | 1.042 | 0.900 | 1.608 | 1.726 |
| E. rhu | 0.726 | 0.793 | 1.138 | 1.015 |
| | | | | |

| 3-Way | | | | |
|---|---|---|---|---|
| PMA | 0.775 | 0.664 | 0.957 | 1.589 |
| BB | 0.992 | 0.883 | 1.702 | 1.962 |
| E. rhu | 1.211 | 0.890 | 1.112 | 1.040 |
| | | | | |

| Non-Vacc. Non-Chall. | | | | |
|---|---|---|---|---|
| PMA | 0.460 | 0.546 | 0.759 | 1.253 |
| BB | 0.921 | 0.637 | 0.778 | 0.668 |
| E. rhu | 0.860 | 0.574 | 0.396 | 0.217 |

| | | | | |
|---|---|---|---|---|
| Comparison to bacterins with PMD and to serum from non-vaccinated pigs to evaluate interference by PMD with those other component organisms. Serums were collected at the time of first vaccination (V1), time of second vaccination (V2), time of challenge (C+2) and time of Necropsy (C+21). Response was measured by ELISA tests with appropriate whole-cell sonicate antigen coated onto the test wells. ^{a} Vaccine groups are as listed in Table 1. The two replicate groups have been combined. | | | | |

### Example 16

### Piglet Vaccination/Challenge Study With and Without Acemannan

A minimum protective dose of PMD as tested was 5 x 10⁸ CFU as determined by reduction of lesions of atrophic rhinitis. Both TAR and TPR values supported this conclusion. A dose of 1 x 10⁹ was also protective as indicated by TPR. There was no striking effect of vaccination or challenge on lung lesions. These observations strongly support the dose of 5 x 10⁸ CFU in the reference bacterin and 1 x 10⁹ CFU in the combination.

No interference with the Pasteurella Type D by Bordetella, Erysipelothrix, and the Type A Pasteurella was reflected in the serologic response to the PMD. However, the lack of significance at the 0.05 level for TPR or TAR in the combination bacterin with 5 x 10⁸ PMD suggests a mild degree of interference. The high dose (3 x 10⁹ CFU) of PMD apparently inhibited the protective response. This is a phenomenon we have seen previously as well. The cause of the high dose interference is uncertain, but the results demand caution in adding excessive (much greater than 1 x 10⁹ CFU) PMD in production serials. Addition of acemannan to the bacterins was not necessary to induce a protective response. In fact, the apparent contribution of this batch of acemannan to vaccination site reactions was sufficient to contraindicate its use.

A benefit of acemannan used seemed to occur in the group given 3 doses at 1, 3, and 5 days of age followed by vaccination with the combination bacterin at 7 and 21 days of age. That group was protected against subsequent PMD challenge to an extent that the TPR was not only significantly improved over non-vaccinated, challenged pigs, but was also the only group whose TPR was not significantly different from the TPR for the non-challenged control group. This observation suggests that a non-specific stimulation or priming can be induced by acemannan in the neonate.

Although the bacterin-toxoids all passed the mouse safety test, some were found to contain low levels of contamination. Acemannan had been added to the bacterin-toxoids as non-sterilized powder and strongly contributed to the contamination found in those products. The formalin concentration for all products was found to be between 0.15 to 0.17%, which is within acceptable limits. If acemannan were to be included in the final formulation, a longer inactivation time would have to be included in the protocol.

In general, evaluation of clinical signs and subjective evaluation or scoring of lung lesions and snout lesions were not useful criteria to determine protection due to vaccination with PMD. The principal lesions of PMD infection is atrophic rhinitis. Thus, it is not surprising that lung lesions did not contribute a major component of the disease in this study. Scoring snouts for severity of atrophic rhinitis is subjective and yields discontinuous data. The application of morphometry in evaluating the snout lesions yields objective data that can be usefully analyzed by parametric statistical methods as done in this study.

### Discussion

The data presented in the above examples suggests that addition of acemannan to a vaccine enhances the immunogenicity of the virus. While the mechanism by which this occurs is not yet certain, one model proposes the involvement of macrophages. All macrophages are known to bear surface mannose receptors, which would be helpful in nonspecific response to bacteria and fungi whose surfaces are often mannose-rich (Largent et al., 1984). It is believed that macrophages may play a role in the transportation and replication of virus in the host following vaccination. The addition of acemannan to the vaccine may aid transformation of resting state macrophages to the activated state, thereby making possible more efficient processing and transport of the virus. This would presumably lead to a more efficacious response to challenge after vaccination, particularly in the case of an early or otherwise serious virus challenge.

The data presented above suggest that a bacterin with a combination of Bordetella, Erysipelothrix and Pasteurella Type A is efficacious in affording protection against disease. The data further suggest that acemannan given parenterally promotes the efficacy of a subsequently administered vaccine.

### References

1. Bacon, L. D. et al. Augmentation of retrovirus-induced lymphoid leukosis by Marek's Disease in White Leghorn Chickens. J. Virol 63: 504-512 (1989).
2. Largent, B. L. et al. Carbohydrate-specific adhesion of alveolar macrophages to mannose-derived surfaces. J. Biol. Chem. 259: 1764-1769 (1984).

## Claims

1. A vaccine comprising an effective immunizing amount, of a modified live virus, a complex carbohydrate in an amount effective to enhance the immunogenicity of the modified live virus and a suitable carrier, wherein said complex carbohydrate is selected from mannan, glucan and levan.

2. The vaccine of claim 1, wherein the modified live virus is a modified live avian virus.

3. The vaccine of claim 2, wherein the modified live avian virus is a modified live Herpesvirus of Turkeys.

4. The vaccine of claim 3, wherein the modified live Herpesvirus of Turkeys is strain FC126 (ATCC No. VR 584B).

5. The vaccine of claim 3 or 4, wherein the effective immunizing amount of the modified live Herpesvirus of Turkeys is present in Herpesvirus of Turkeys-infected cells.

6. The vaccine of any one of claims 3 to 5, wherein the effective immunizing amount of the modified live Herpesvirus of Turkeys is the amount present in about 20,000 Herpesvirus of Turkeys-infected cells.

7. The vaccine of claim 2, wherein the modified live avian virus is a modified live Marek's Disease Virus, a modified live Newcastle Disease Virus, a modified live Infectious Bursal Disease Virus, a modified live Infectious Bronchitis Virus or a modified live Avian Reovirus.

8. The vaccine of claim 1, wherein the modified live virus is a modified live swine virus.

9. The vaccine of claim 8, wherein the modified live swine virus is a modified live pseudorabies virus, a modified live coronavirus or a modified live parvovirus.

10. The vaccine of claim 1, wherein the modified live virus is a modified live feline virus.

11. The vaccine of claim 10, wherein the modified live feline virus is a modified live Feline Leukemia Virus, a modified live Feline T-cell Leukemia Virus or a modified live Feline Immunodeficiency Virus.

12. The vaccine of claim 1, wherein the modified live virus is a modified live canine virus.

13. The vaccine of claim 12, wherein the modified live canine virus is a modified live coronavirus, a modified live parvovirus or a modified live distemper virus.

14. The vaccine of claim 1, further comprising a second modified live virus.

15. The vaccine of claim 14, wherein the first and second modified live viruses are modified live avian viruses.

16. The vaccine of claim 15, wherein the first modified live avian virus is a modified live Herpesvirus of Turkeys and the second modified live avian virus is a modified live Marek's Disease Virus serotype I, or a modified live Marek's Disease Virus serotype II, or a modified live Newcastle Disease Virus, or a modified live Infectious Bursal Disease Virus, or a modified live Infectious Bronchitis Virus, or a modified live Avian Reovirus.

17. The vaccine of claim 15, wherein the first modified live avian virus is a modified live Marek's Disease Virus serotype I and the second modified live avian virus is a modified live Marek's Disease Virus serotype II, or a modified live Newcastle Disease Virus, or a modified live Infectious Bursal Disease Virus, or a modified live Infectious Bronchitis Virus, or a modified live Avian Reovirus.

18. The vaccine of claim 15, wherein the first modified live avian virus is a modified live Marek's Disease Virus serotype II and the second modified live avian virus is a modified live Newcastle Disease Virus, or a modified live Infectious Bursal Disease Virus, or a modified live Infectious Bronchitis Virus, or a modified live Avian Reovirus.

19. The vaccine of claim 15, wherein the first modified live avian virus is a modified live Newcastle Disease Virus and the second modified live avian virus is a modified live infectious Bursal Disease Virus, or a modified live Infectious Bronchitis Virus, or a modified live Avian Reovirus.

20. The vaccine of claim 15, wherein the first modified live avian virus is a modified live Infectious Bursal Disease Virus and the second modified live avian virus is a modified live Infectious Bronchitis Virus, or a modified live Avian Reovirus.

21. The vaccine of claim 15, wherein the first modified live avian virus is a modified live Infectious Bronchitis Virus and the second modified live avian virus is a modified live Avian Reovirus.

22. The vaccine of claim 14, wherein the first and second modified live viruses are modified live swine viruses.

23. The vaccine of claim 14, wherein the first and second modified live viruses are modified live feline viruses.

24. The vaccine of claim 14, wherein the first and second modified live viruses are modified live canine viruses.

25. The vaccine of claim 14, further comprising a third modified live virus.

26. The vaccine of any one of claims 1 to 25, wherein the effective immunizing amount of the modified live virus is an amount greater than about 1,000 plaque forming units.

27. The vaccine of any one of claims 1 to 6, 14 to 20 and 25 to 26, wherein the effective immunizing amount of the modified live Herpesvirus of Turkeys is an amount greater than about 3,000 plaque forming units.

28. The vaccine of claim 1, wherein the mannan is a β(1 → 4) linked mannan.

29. The vaccine of claim 28, wherein the β(1 → 4) linked mannan is an acemannan.

30. The vaccine of claim 29, wherein the acemannan is an extract from the leaf of the Aloe barbadensis plant.

31. The vaccine of claim 29 or 30, wherein the effective amount of the acemannan is an amount greater than about 50 micrograms.

32. The vaccine of claim 31, wherein the effective amount of the acemannan is an amount from about 50 micrograms to about 1,000 micrograms, preferably an amount from about 50 micrograms to about 150 micrograms, and most preferably an amount of about 100 micrograms.

33. The vaccine of any one of claims 1 to 32, wherein the suitable carrier comprises an aqueous buffer.

34. The vaccine of claim 33, wherein the aqueous buffer comprises a phosphate buffer.

35. Use of a modified live virus, a complex carbohydrate and a suitable carrier for the preparation of a vaccine of any one of claims 1 to 34 for immunizing an animal against viral disease.

36. Use of claim 35, wherein the animal is a day of age fowl, a porcine, feline or canine.

37. Use of claim 36, wherein the day of age fowl is a day of age chicken, turkey, duck or quail.

38. Use of any one of claims 35 to 37, wherein the immunization comprises intramuscular injection, subcutaneous injection, oral administration or administration by eye drops.

39. Use of claim 35, wherein the animal is a chick and the disease is Marek's Disease.

## Patentansprüche

1. Impfstoff, umfassend eine wirksame immunisierende Menge eines modifizierten Lebendvirus, ein komplexes Kohlenhydrat in einer Menge, die wirksam ist, um die Immunogenität des modifizierten Lebendvirus zu verstärken, und einen geeigneten Träger, wobei das komplexe Kohlenhydrat ausgewählt ist aus Mannan, Glucan und Lävan.

2. Impfstoff nach Anspruch 1, wobei das modifizierte Lebendvirus ein modifiziertes Lebend-Vogelvirus ist.

3. Impfstoff nach Anspruch 2, wobei das modifizierte Lebend-Vogelvirus ein modifiziertes Lebend-Putenherpesvirus ist.

4. Impfstoff nach Anspruch 3, wobei das modifizierte Lebend-Putenherpesvirus der Stamm FC126 (ATCC Nr. VR 584B) ist.

5. Impfstoff nach Anspruch 3 oder 4, wobei die wirksame immunisierende Menge des modifizierten Lebend-Putenherpesvirus in von Putenherpesvirus infizierten Zellen vorhanden ist.

6. Impfstoff nach einem der Ansprüche 3 bis 5, wobei die wirksame immunisierende Menge des modifizierten Lebend-Putenherpesvirus die Menge ist, welche in etwa 20.000 Puten-Herpesvirus-infizierten Zellen vorhanden ist.

7. Impfstoff nach Anspruch 2, wobei das modifizierte Lebend-Vogelvirus ein modifiziertes Lebend-Marek-Virus, ein modifiziertes Lebend-Newcastle-Disease-Virus, ein modifiziertes Lebend-Infectious-Bursal-Disease-Virus, ein modifiziertes Lebend-Infectious Bronchitis Virus oder ein modifiziertes Lebend-Vogel-Reovirus ist.

8. Impfstoff nach Anspruch 1, wobei das modifizierte Lebendvirus ein modifiziertes Lebend-Schweinevirus ist.

9. Impfstoff nach Anspruch 8, wobei das modifizierte Lebend-Schweinevirus ein modifiziertes Lebend-Pseudowut-Virus, ein modifiziertes Lebend-Coronavirus oder ein modifiziertes Lebend-Parvovirus ist.

10. Impfstoff nach Anspruch 1, wobei das modifizierte Lebendvirus ein modifiziertes Lebend-Katzenvirus ist.

11. Impfstoff nach Anspruch 10, wobei das modifizierte Lebend-Katzenvirus ein modifiziertes Lebend-Katzenleukämievirus, ein modifiziertes Lebend-Katzen-T-Zell-Leukämievirus oder ein modifiziertes Lebend-Katzen-Immundefektvirus ist.

12. Impfstoff nach Anspruch 1, wobei das modifizierte Lebendvirus ein modifiziertes Lebend-Hundevirus ist.

13. Impfstoff nach Anspruch 12, wobei das modifizierte Lebend-Hundevirus ein modifiziertes Lebend-Coronavirus, ein modifiziertes Lebend-Parvovirus oder ein modifiziertes Lebend-Staupe-Virus ist.

14. Impfstoff nach Anspruch 1, weiterhin umfassend ein zweites modifiziertes Lebendvirus.

15. Impfstoff nach Anspruch 14, wobei die ersten und zweiten modifizierten Lebend-Viren modifizierte Lebend-Vogelviren sind.

16. Impfstoff nach Anspruch 15, wobei das erste modifizierte Lebend-Vogelvirus ein modifiziertes Lebend-Putenherpesvirus ist und das zweite modifizierte Lebend-Vogelvirus ein modifiziertes Lebend-Marek-Virus Serotyp I oder ein modifiziertes Lebend-Marek-Virus Serotyp II oder ein modifiziertes Lebend-Newcastle-Disease-Virus, oder ein modifiziertes Lebend-Infectious-Bursal-Disease-Virus oder ein modifiziertes Lebend-Infectious-Bronchitis-Virus oder ein modifiziertes Lebend-Vogel-Reovirus ist.

17. Impfstoff nach Anspruch 15, wobei das erste modifizierte Lebend-Vogelvirus ein modifiziertes Lebend-Marek-Virus Serotyp I und das zweite modifizierte Lebend-Vogelvirus ein modifiziertes Lebend-Marek-Virus Serotyp II oder ein modifiziertes Lebend-Newcastle-Disease-Virus oder ein modifiziertes Lebend-Infectious-Bursal-Disease-Virus oder ein modifiziertes Lebend-Infectious-Bronchitis-Virus oder ein modifiziertes Lebend-Vogel-Reovirus ist.

18. Impfstoff nach Anspruch 15, wobei das erste modifizierte Lebend-Vogelvirus ein modifiziertes Lebend-Marek-Virus Serotyp II und das zweite modifizierte Lebend-Vogelvirus ein modifiziertes Lebend-Newcastle-Disease-Virus oder ein modifiziertes Lebend-Infectious-Bursal-Disease-Virus oder ein modifiziertes Lebend-Infectious-Bronchitis-Virus oder ein modifiziertes Lebend-Vogel-Reovirus ist.

19. Impfstoff nach Anspruch 15, wobei das erste modifizierte Lebend-Vogelvirus ein modifiziertes Lebend-Newcastle-Disease-Virus und das zweite modifizierte Lebend-Vogelvirus ein modifiziertes Lebend-Infectious-Bursal-Disease-Virus oder ein modifiziertes Lebend-Infectious-Bronchitis-Virus oder ein modifiziertes Lebend-Vogel-Reovirus ist.

20. Impfstoff nach Anspruch 15, wobei das erste modifizierte Lebend-Vogelvirus ein modifiziertes Lebend-Infectious-Bursal-Disease-Virus und das zweite modifizierte Lebend-Vogelvirus ein modifiziertes Lebend-Infectious-Bronchitis-Virus oder ein modifiziertes Lebend-Vogel Reovirus ist.

21. Impfstoff nach Anspruch 15, wobei das erste modifizierte Lebend-Vogelvirus ein modifiziertes Lebend-Infectious-Bronchitis-Virus und das zweite modifizierte Lebend-Vogelvirus ein modifiziertes Lebend-Vogel-Reovirus ist.

22. Impfstoff nach Anspruch 14, wobei die ersten und zweiten modifizierten Lebend-Viren modifizierte Lebend-Schweineviren sind.

23. Impfstoff nach Anspruch 14, wobei die ersten und zweiten modifizierten Lebend-Viren modifizierte Lebend-Katzenviren sind.

24. Impfstoff nach Anspruch 14, wobei die ersten und zweiten modifizierten Lebend-Viren modifizierte Lebend-Hundeviren sind.

25. Impfstoff nach Anspruch 14, weiterhin umfassend ein drittes modifiziertes Lebendvirus.

26. Impfstoff nach einem der Ansprüche 1 bis 25, wobei die wirksame immunisierende Menge des modifizierten Lebendvirus eine Menge von größer als etwa 1.000 Plaque-bildende Einheiten ("plaque forming units") ist.

27. Impfstoff nach einem der Ansprüche 1 bis 6, 14 bis 20 und 25 bis 26, wobei die wirksame immunisierende Menge des modifizierten Lebend-Putenherpesvirus eine Menge größer als etwa 3.000 Plaque-bildende Einheiten ("plaque forming units") ist.

28. Impfstoff nach Anspruch 1, wobei das Mannan ein β(1 → 4) verknüpftes Mannan ist.

29. Impfstoff nach Anspruch 28, wobei das β(1 → 4) verknüpfte Mannan ein Acemannan ist.

30. Impfstoff nach Anspruch 29, wobei das Acemannan ein Extrakt aus dem Blatt der Aloe barbadensis-Pflanze ist.

31. Impfstoff nach Anspruch 29 oder 30, wobei der wirksame Teil des Acemannan eine Menge größer als etwa 50 Mikrogramm ist.

32. Impfstoff nach Anspruch 31, wobei die wirksame Menge des Acemannan eine Menge von etwa 50 Mikrogramm bis etwa 1000 Mikrogramm, vorzugsweise eine Menge von etwa 50 Mikrogramm bis etwa 150 Mikrogramm und besonders bevorzugt eine Menge von etwa 100 Mikrogramm ist.

33. Impfstoff nach einem der Ansprüche 1 bis 32, wobei der geeignete Träger einen wässrigen Puffer umfasst.

34. Impfstoff nach Anspruch 33, wobei der wässrige Puffer einen Phosphatpuffer umfasst.

35. Verwendung eines modifizierten Lebendvirus, eines komplexen Kohlenhydrats und eines geeigneten Trägers für die Herstellung eines Impfstoffs nach einem der Ansprüche 1 bis 34 zur Immunisierung eines Tiers gegen Viruskrankheiten.

36. Verwendung nach Anspruch 35, wobei das Tier ein Tag altes Geflügel, ein Schwein, eine Katze oder ein Hund ist.

37. Verwendung nach Anspruch 36, wobei das ein Tag alte Geflügel ein ein Tag altes Küken, eine ein Tag alte Pute, Ente oder Wachtel ist.

38. Verwendung nach einem der Ansprüche 35 bis 37, wobei die Immunisierung intramuskuläre Injektion, subkutane Injektion, orale Verabreichung oder Verabreichung durch Augentropfen umfasst.

39. Verwendung nach Anspruch 35, wobei das Tier ein Huhn und die Krankheit Marek's Disease ist.

## Revendications

1. Vaccin comprenant une quantité immunisante efficace d'un virus vivant modifié, un glucide complexe en une quantité efficace pour augmenter l'immunogénicité du virus vivant modifié et un support adapté, dans lequel ledit glucide complexe est sélectionné parmi un mannane, un glycane, et un lévane.

2. Vaccin selon la revendication 1, dans lequel le virus vivant modifié est un virus aviaire vivant modifié.

3. Vaccin selon la revendication 2, dans lequel le virus aviaire vivant modifié est un virus de l'Herpès de dinde vivant modifié.

4. Vaccin selon la revendication 3, dans lequel le virus de l'Herpès de dinde vivant modifié est la souche FC126 (ATCC No. VR 584B).

5. Vaccin selon la revendication 3 ou 4, dans lequel la quantité immunisante efficace du virus de l'Herpès de dinde vivant modifié est présente dans des cellules infectées par un virus de l'Herpès de dinde.

6. Vaccin selon l'une quelconque des revendications 3 à 5, dans lequel la quantité immunisante efficace du virus de l'Herpès de dinde vivant modifié est la quantité présente dans environ 20 000 cellules infectées par un virus de l'Herpès de dinde.

7. Vaccin selon la revendication 2, dans lequel le virus aviaire vivant modifié est un virus vivant modifié de la Maladie de Marek, un virus vivant modifié de la maladie de Newcastle, un virus vivant modifié de la Bursite Infectieuse, un virus vivant modifié de la bronchite infectieuse, ou un réovirus aviaire vivant modifié.

8. Vaccin selon la revendication 1, dans lequel le virus vivant modifié est un virus vivant modifié de porc.

9. Vaccin selon la revendication 8, dans lequel le virus vivant modifié de porc est un virus de la pseudo-rage vivant modifié, un coronavirus vivant modifié ou un parvovirus vivant modifié.

10. Vaccin selon la revendication 1, dans lequel le virus vivant modifié est un virus vivant modifié félin.

11. Vaccin selon la revendication 10, dans lequel le virus vivant modifié félin est un virus de la Leucémie Féline vivant modifié, un virus de la Leucémie des Cellules T Féline vivant modifié, ou un virus de l'Immunodéficience Féline vivant modifié.

12. Vaccin selon la revendication 1, dans lequel le virus vivant modifié est un virus vivant modifié canin.

13. Vaccin selon la revendication 12, dans lequel le virus vivant modifié canin est un coronavirus vivant modifié, un parvovirus vivant modifié ou un virus de la maladie de Carré vivant modifié.

14. Vaccin selon la revendication 1, comprenant en outre un deuxième virus vivant modifié.

15. Vaccin selon la revendication 14, dans lequel le premier et le deuxième virus vivants modifiés sont des virus aviaires vivants modifiés.

16. Vaccin selon la revendication 15, dans lequel le premier virus aviaire vivant modifié est un virus de l'Herpès de dinde vivant modifié et le deuxième virus aviaire vivant modifié est un virus vivant modifié de la Maladie de Marek de Sérotype I, ou un virus vivant modifié de la Maladie de Marek de Sérotype II, ou un virus vivant modifié de la maladie de Newcastle, ou un virus vivant modifié de la bursite infectieuse, ou un virus vivant modifié de la bronchite infectieuse, ou un Réovirus aviaire vivant modifié.

17. Vaccin selon la revendication 15, dans lequel le premier virus aviaire vivant modifié est un virus vivant modifié de la maladie de Marek de sérotype I et le deuxième virus aviaire vivant modifié est un virus vivant modifié de la maladie de Marek de sérotype II, ou un virus vivant modifié de la maladie de Newcastle, ou un virus vivant modifié de la bursite infectieuse, ou un virus vivant modifié de la bronchite infectieuse, ou un Réovirus aviaire vivant modifié.

18. Vaccin selon la revendication 15, dans lequel le premier virus aviaire vivant modifié est un virus vivant modifié de la maladie de Marek de sérotype II et le deuxième virus aviaire vivant modifié est un virus vivant modifié de la maladie de Newcastle, ou un virus vivant modifié de la bursite infectieuse, ou un virus vivant modifié de la bronchite infectieuse, ou un Réovirus aviaire vivant modifié.

19. Vaccin selon la revendication 15, dans lequel le premier virus aviaire vivant modifié est un virus vivant modifié de la maladie de Newcastle et le deuxième virus aviaire vivant modifié est un virus vivant modifié de la bursite infectieuse, ou un virus vivant modifié de la bronchite infectieuse, ou un réovirus aviaire vivant modifié.

20. Vaccin selon la revendication 15, dans lequel le premier virus aviaire vivant modifié est un virus vivant modifié de la bursite infectieuse et le deuxième virus aviaire vivant modifié est un virus vivant modifié de la bronchite infectieuse, ou un Réovirus aviaire vivant modifié.

21. Vaccin selon la revendication 15, dans lequel le premier virus aviaire vivant modifié est un virus vivant modifié de la bronchite infectieuse et le deuxième virus aviaire vivant modifié est un Réovirus aviaire vivant modifié.

22. Vaccin selon la revendication 14, dans lequel le premier et le deuxième virus vivants modifiés sont des virus vivants modifiés de porc.

23. Vaccin selon la revendication 14, dans lequel le premier et le deuxième virus vivants modifiés sont des virus vivants modifiés félins.

24. Vaccin selon la revendication 14, dans lequel le premier et le deuxième virus vivants modifiés sont des virus vivants modifiés canins.

25. Vaccin selon la revendication 14, comprenant en outre un troisième virus vivant modifié.

26. Vaccin selon l'une quelconque des revendications 1 à 25, dans lequel la quantité immunisante efficace de virus vivant modifié est une quantité supérieure à environ 1000 pfu (Unité Formant Plaque).

27. Vaccin selon l'une quelconque des revendications 1 à 6, 14 à 20 et 25 à 26, dans lequel la quantité immunisante efficace de virus de l'Herpès de dinde vivant modifié est une quantité supérieure à environ 3000 pfu.

28. Vaccin selon la revendication 1, dans lequel le mannane est un mannane à liaison β(1 → 4).

29. Vaccin selon la revendication 28, dans lequel le mannane à liaison β(1 → 4) est un acémannane.

30. Vaccin selon la revendication 29, dans lequel l'acémannane est un extrait de feuille de plante Aloe barbadensis.

31. Vaccin selon la revendication 29 ou 30, dans lequel la quantité efficace d'acémannane est une quantité supérieure à environ 50 microgrammes.

32. Vaccin selon la revendication 31, dans lequel la quantité efficace d'acémannane est une quantité d'environ 50 microgrammes à environ 1000 microgrammes, de préférence une quantité d'environ 50 microgrammes à environ 150 microgrammes, et tout préférentiellement une quantité d'environ 100 microgrammes.

33. Vaccin selon l'une quelconque des revendications 1 à 32, dans lequel le support adapté comprend un tampon aqueux.

34. Vaccin selon la revendication 33, dans lequel le tampon aqueux comprend un tampon phosphate.

35. Utilisation d'un virus vivant modifié, d'un glucide complexe et d'un support adapté pour la préparation d'un vaccin selon l'une quelconque des revendications 1 à 34 pour immuniser un animal contre une maladie virale.

36. Utilisation selon la revendication 35, dans laquelle l'animal est une volaille d'un jour, un porcin, un félin ou un canin.

37. Utilisation selon la revendication 36, dans laquelle la volaille d'un jour est un poulet, une dinde, un canard ou une caille d'un jour.

38. Utilisation selon l'une quelconque des revendications 35 à 37, dans laquelle l'immunisation comprend une injection intramusculaire, une injection sous-cutanée, une administration orale, ou une administration par des gouttes ophtalmiques.

39. Utilisation selon la revendication 35, dans laquelle l'animal est un poussin et la maladie est la Maladie de Marek.
